# EUROPEAN PATENT APPLICATION

(11) **EP 4 159 759 A1**
(43) Date of publication of application: **05.04.2023**
(21) Application number: 21816925.8
(22) Date of filing: 02.06.2021
(51) Int. Cl.: C07K 16/28, A61K 47/68, A61K 39/395, A61P 35/00

(54) **ANTI-B7-H3 ANTIBODY AND PREPARATION THEREFOR AND USE THEREOF**

(30) Priority: 02.06.2020 CN 202010491448
(71) Applicant: Minghui Pharmaceutical (Shanghai) Limited, Pilot Free Trade Zone Pudong New Area Shanghai 201203 (CN); Minghui Pharmaceutical (Hangzhou) Limited, Hangzhou, Zhejiang 310018 (CN)
(72) Inventor: ZHU, Liyuan, Shanghai 201203 (CN); WANG, Wenyi, Shanghai 201203 (CN); LANG, Guojun, Shanghai 201203 (CN); DENG, Min, Shanghai 201203 (CN)
(74) Representative: Lavoix
(86) International application number: PCT/CN2021/098018
(87) International publication number: WO 2021/244590

(57) **Abstract**

Provided are an anti-B7-H3 antibody and preparation thereof and a use thereof. Further provided is a drug conjugate and recombinant protein containing the antibody. The antibody of the present invention can be effectively endocytosed by cells, and an antibody-conjugated drug prepared by using the antibody of the present invention shows a tumor inhibition effect.

## Description

### Technical Field

The present invention relates to the biomedical field, in particular to an anti-B7-H3 antibody as well as preparation and use thereof.

### Background

The growth and metastasis of tumors depend to a large extent on their capacity to evade host immune surveillance and overcome host defenses. Most tumors express antigens that can be recognized to a variable extent by the host immune system, but in many cases, an inadequate immune response is elicited because of the ineffective activation of effector T cells.

CD4+ T-lymphocytes are the essential organizers of most mammalian immune and autoimmune responses. The activation of CD4+ helper T-cells has been found to be mediated through co-stimulatory interactions between antigen presenting cells and naive CD4+ T-lymphocytes. Two kinds of interactions between cells are required in this process. In the first kind of interaction, an antigen presenting cell must display the relevant target antigen on cell surface through the major histocompatibility complex so that it can bind to the T-cell Receptor ("TCR") of a naive CD4+ T-lymphocyte. In the second kind of interaction, a ligand of the antigen presenting cell must bind to a CD28 receptor on the surface of CD4+ T-lymphocyte. After experiencing the co-stimulatory signals, CD4+ helper T-cells are then capable of responding to cytokines (such as Interleukin-2 and Interleukin-12) to develop into Th1 cells. Such cells can produce interferon-gamma (IFN-γ) and tumor necrosis factor-alpha (TNF-α), which further mediate inflammatory responses to target cells expressing the target antigen. B-cell activation and proliferation also occurs, resulting in antibody production specific for the target antigen. During the activation of T cells, the absence of any one of both co-stimulatory signals will cause T cells to enter the state of clonal anergy. In pathologic states, Th1 cells are the key players of various organ specific autoimmune diseases, such as type I diabetes, rheumatoid arthritis, and multiple sclerosis.

B7 family members are immunoglobulin super family members with an immunoglobulin-V-like and an immunoglobulin-C-like domain (e.g., IgV-IgC). The IgV and IgC domains of B7-family members are each encoded by a single exon, with additional exons encoding leader sequences, transmembrane domain and cytoplasmic domain. The cytoplasmic domain is short, ranging in length from 19 to 62 amino acid residues and can be encoded by multiple exons.

B7-H3 is unique in that the major human form thereof contains two extracellular tandem IgV-IgC domains (i.e., IgV-IgC-IgV-IgC). Members of the B7 family are predicted to form back-to-back, non-covalent homodimers on the cell surface, and such dimers have been found with respect to B7-1 (CD80) and B7-2 (CD86). A four immunoglobulin extracellular domain variant ("41g-B7-H3") has been identified and found to be more common human protein form. Since the natural murine form (2Ig) and the human 4Ig form exhibit similar function, no functional difference has been observed between these two forms. The 41g-B7-H3 molecule inhibits the natural killer cell-mediated lysis of cancer cells. The human B7-H3 (2Ig form) has been found to promote T-cell activation and IFN-γ production by binding to a specific receptor on activated T cells.

When B7-H3 mediates both T cell co-stimulation and co-inhibition, the acting mode of the protein is complex. B7-H3 binds to (TREM)-like transcript 2 (TLT-2) and co-stimulates T cell activation, and binds to as yet unidentified receptors to mediate co-inhibition of T cells. In addition, through interactions with unknown receptors, B7-H3 has become an inhibitor for natural killer cells and osteoblastic cells. Such inhibition may operate through interactions with members of the most signaling pathways through which T cell receptor (TCR) regulates gene transcription (e.g., NFTA, NF-κB or AP-1 factor). B7-H3 co-stimulates CD4+ and CD8+ T-cell proliferation. B7-H3 also stimulates IFN-γ production and CD8+ lytic activity. However, the protein also may inhibit T-cell activation through NFAT (nuclear factor of activated T-cells), NF-KB (nuclear factor kappa B), and AP-1 (activator protein-1) factor. B7-H3 is also believed to inhibit Th1, Th2, or Th17 *in vivo.*

Several independent studies have shown that human malignant tumor cells exhibit a significant increase in expression of B7-H3 protein and that such increased expression is associated with increased disease severity, indicating that B7-H3 is exploited by tumors as an immune evasion pathway. In addition to its expression on neuroblastoma cells, human B7-H3 is also known to be expressed on a variety of other cancer cells (e.g., gastric cancer, ovarian cancer and non-small cell lung cancer). Blocking the binding of B7-H3 to its receptor, or developing corresponding targeted therapeutic products by using B7-H3 as a high-expression antigen in tumors, are potential means to treat various tumors.

At present, there are still many deficiencies in tumor-targeting therapeutic products developed targeting B7-H3 in the prior art and there is a need to develop new therapeutic antibody related products in this field.

### Summary of the invention

The purpose of the present invention is to provide an anti-B7-H3 antibody and preparation thereof and use thereof.

In the first aspect of the present invention, it provides a heavy chain variable region of an antibody having the following three complementarity determining regions (CDRs):
a VH-CDR1 as shown in SEQ ID NO: 3n,
a VH-CDR2 as shown in SEQ ID NO: 3n+1, and
a VH-CDR3 as shown in SEQ ID NO: 3n+2;
wherein each n is independently 11, 12, 13, 14, 15, 16, or 17;
or, wherein the heavy chain variable region comprises the following three complementarity determining regions (CDRs):
a VH-CDR1 as shown in SEQ ID NO: 97,
a VH-CDR2 as shown in SEQ ID NO: 54, and
a VH-CDR3 as shown in SEQ ID NO: 35;
or
a VH-CDR1 as shown in SEQ ID NO: 33,
a VH-CDR2 as shown in SEQ ID NO: 54, and
a VH-CDR3 as shown in SEQ ID NO: 35;
or
a VH-CDR1 as shown in SEQ ID NO: 36,
a VH-CDR2 as shown in SEQ ID NO: 55, and
a VH-CDR3 as shown in SEQ ID NO: 56;
or
a VH-CDR1 as shown in SEQ ID NO: 57,
a VH-CDR2 as shown in SEQ ID NO: 40, and
a VH-CDR3 as shown in SEQ ID NO: 41;
or
a VH-CDR1 as shown in SEQ ID NO: 51,
a VH-CDR2 as shown in SEQ ID NO: 52, and
a VH-CDR3 as shown in SEQ ID NO: 58;
or
a VH-CDR1 as shown in SEQ ID NO: 51,
a VH-CDR2 as shown in SEQ ID NO: 59, and
a VH-CDR3 as shown in SEQ ID NO: 60;
wherein any one of the above amino acid sequences further includes a derivative sequence that is optionally with at least one amino acid added, deleted, modified, and/or substituted, and can retain the binding affinity to B7-H3.

In another preferred embodiment, the heavy chain variable region comprises the following three complementarity determining regions (CDRs):

| VH-CDR1 Sequence Number | VH-CDR2 Sequence Number | VH-CDR3 Sequence Number |
|---|---|---|
| 33 | 34 | 35 |
| 33 | 54 | 35 |
| 97 | 54 | 35 |
| 36 | 37 | 38 |
| 36 | 55 | 56 |
| 39 | 40 | 41 |
| 57 | 40 | 41 |
| 42 | 43 | 44 |
| 45 | 46 | 47 |
| 48 | 49 | 50 |
| 51 | 52 | 53 |
| 51 | 52 | 58 |
| 51 | 59 | 60. |

In another preferred embodiment, the heavy chain variable region has an amino acid sequence as shown in any one of SEQ ID NOs: 79-96.

In the second aspect of the present invention, it provides a heavy chain of an antibody having the heavy chain variable region according to the first aspect of the present invention.

In another preferred embodiment, the heavy chain further comprises a heavy chain constant region.

In another preferred embodiment, the heavy chain constant region is of human origin.

In another preferred embodiment, the heavy chain constant region is a human antibody heavy chain IgG1 or IgG4 constant region.

In the third aspect of the present invention, it provides a light chain variable region of an antibody having the following three complementarity determining regions (CDRs):
a VL-CDR1 as shown in SEQ ID NO: 3m+1,
a VL-CDR2 as shown in SEQ ID NO: 3m+2, and
a VL-CDR3 as shown in SEQ ID NO: 3m+3;
wherein each m is independently 0, 1, 2, 3, 4, 5, 6, or 7;
or, wherein the light chain variable region comprises the following three complementarity determining regions (CDRs):
a VL-CDR1 as shown in SEQ ID NO: 1,
a VL-CDR2 as shown in SEQ ID NO: 25, and
a VL-CDR3 as shown in SEQ ID NO: 3;
or
a VL-CDR1 as shown in SEQ ID NO: 1,
a VL-CDR2 as shown in SEQ ID NO: 26, and
a VL-CDR3 as shown in SEQ ID NO: 3;
or
a VL-CDR1 as shown in SEQ ID NO: 27,
a VL-CDR2 as shown in SEQ ID NO: 5, and
a VL-CDR3 as shown in SEQ ID NO: 6;
or
a VL-CDR1 as shown in SEQ ID NO: 27,
a VL-CDR2 as shown in SEQ ID NO: 28, and
a VL-CDR3 as shown in SEQ ID NO: 6;
or
a VL-CDR1 as shown in SEQ ID NO: 29,
a VL-CDR2 as shown in SEQ ID NO: 11, and
a VL-CDR3 as shown in SEQ ID NO: 12;
or
a VL-CDR1 as shown in SEQ ID NO: 30,
a VL-CDR2 as shown in SEQ ID NO: 23, and
a VL-CDR3 as shown in SEQ ID NO: 31;
or
a VL-CDR1 as shown in SEQ ID NO: 30,
a VL-CDR2 as shown in SEQ ID NO: 23, and
a VL-CDR3 as shown in SEQ ID NO: 32;
wherein any one of the above amino acid sequences further includes a derivative sequence that is optionally with at least one amino acid added, deleted, modified, and/or substituted, and can retain the binding affinity to B7-H3.

In another preferred embodiment, the light chain variable region comprises the following three complementarity determining regions (CDRs):

| VL-CDR1 Sequence Number | VL-CDR2 Sequence Number | VL-CDR3 Sequence Number |
|---|---|---|
| 1 | 2 | 3 |
| 1 | 25 | 3 |
| 1 | 26 | 3 |
| 4 | 5 | 6 |
| 27 | 5 | 6 |
| 27 | 28 | 6 |
| 7 | 8 | 9 |
| 10 | 11 | 12 |
| 29 | 11 | 12 |
| 13 | 14 | 15 |
| 16 | 17 | 18 |
| 19 | 20 | 21 |
| 22 | 23 | 24 |
| 30 | 23 | 31 |
| 30 | 23 | 32. |

In another preferred embodiment, the light chain variable region has an amino acid sequence as shown in any one of SEQ ID NOs: 61 -78.

In the fourth aspect of the present invention, it provides a light chain of an antibody having the light chain variable region according to the third aspect of the present invention.

In another preferred embodiment, the light chain further comprises a light chain constant region.

In another preferred embodiment, the light chain constant region is of human origin.

In another preferred embodiment, the light chain constant region is a human antibody light chain kappa constant region.

In the fifth aspect of the present invention, it provides an antibody having:
(1) the heavy chain variable region according to the first aspect of the present invention; and/or
(2) the light chain variable region according to the third aspect of the present invention;
   or the antibody has the heavy chain according to the second aspect of the present invention; and/or the light chain according to the fourth aspect of the present invention,
   wherein any one of the above amino acid sequences further includes a derivative sequence that is optionally with at least one amino acid added, deleted, modified, and/or substituted, and can retain the binding affinity to B7-H3.

In another preferred embodiment, the amino acid sequence of any of the above-mentioned CDRs contains a derivative CDR sequence of 1, 2, or 3 amino acids that has been added, deleted, modified, and/or substituted, and so that the derivative antibody composed of VH and VL containing the derivative CDR sequence can retain the affinity for binding to B7-H3.

In another preferred embodiment, the ratio (F1/F0) of the affinity of the derivative antibody binding to B7-H3 (F1) and the affinity of the corresponding non-derivative antibody binding to B7-H3 (F0) is 0.5-2, preferably 0.7-1.5, and more preferably 0.8-1.2.

In another preferred embodiment, the number of added, deleted, modified and/or substituted amino acids is 1-5 (such as 1-3, preferably 1-2, more preferably 1).

In another preferred embodiment, the derivative sequence that is with at least one amino acid added, deleted, modified and/or substituted and can retain B7-H3 binding affinity is an amino acid sequence with homology or sequence identity of at least 96%.

In another preferred embodiment, the antibody further comprises a heavy chain constant region and/or light chain constant region.

In another preferred embodiment, the heavy chain constant region is of human origin, and/or the light chain constant region is of human origin.

In another preferred embodiment, the heavy chain constant region is a human antibody heavy chain IgG1 or IgG4 constant region, and the light chain constant region is a human antibody light chain kappa constant region.

In another preferred embodiment, the antibody is selected from the group consisting of an animal-derived antibody, chimeric antibody, humanized antibody, fully human antibody, and a combination thereof.

In another preferred embodiment, the ratio (Z1/Z0) of the immunogenicity of the chimeric antibody in humans (Z1) and the immunogenicity of non-chimeric antibody (such as murine antibodies) in humans (Z0) is 0-0.5, preferably 0 -0.2, more preferably 0-0.05 (e.g., 0.001-0.05).

In another preferred embodiment, the antibody is a partially or fully humanized, or fully human monoclonal antibody.

In another preferred embodiment, the antibody is a double-chain antibody or a single-chain antibody.

In another preferred embodiment, the antibody is a full-length protein of an antibody, or an antigen binding fragment.

In another preferred embodiment, the antibody is a bispecific antibody or a multispecific antibody.

In another preferred embodiment, the antibody has one or more characteristics selected from the group consisting of:
(a) inhibiting the migration or metastasis of tumor cells;
(b) inhibiting tumor growth; and
(c) relieving tumor immunosuppression of T cells.

In another preferred embodiment, the antibody has the heavy chain variable region according to the first aspect of the present invention and the light chain variable region according to the third aspect of the present invention;
wherein the heavy chain variable region and the light chain variable region are as follows:

| VH Sequence Number | VL Sequence Number |
|---|---|
| 79 | 61 |
| 83 | 64 |
| 83 | 62 |
| 81 | 63 |
| 81 | 62 |
| 82 | 64 |
| 80 | 62 |
| 82 | 62 |
| 84 | 65 |
| 86 | 67 |
| 86 | 69 |
| 86 | 68 |
| 85 | 69 |
| 85 | 66 |
| 85 | 68 |
| 87 | 68 |
| 87 | 69 |
| 88 | 70 |
| 89 | 71 |
| 90 | 72 |
| 91 | 73 |
| 92 | 74 |
| 93 | 75 |
| 94 | 76 |
| 95 | 77 |
| 96 | 78 |

wherein any one of the above amino acid sequences further includes a derivative sequence that is optionally with at least one amino acid added, deleted, modified, and/or substituted, and can retain the binding affinity to B7-H3.

In another preferred embodiment, the antibody has the heavy chain variable region according to the first aspect of the present invention and the light chain variable region according to the third aspect of the present invention;
wherein the heavy chain variable region comprises the following three complementarity determining regions (CDRs):
a VH-CDR1 as shown in SEQ ID NO: 97,
a VH-CDR2 as shown in SEQ ID NO: 54, and
a VH-CDR3 as shown in SEQ ID NO: 35;
the light chain variable region comprises the following three complementarity determining regions (CDRs):
a VL-CDR1 as shown in SEQ ID NO: 1,
a VL-CDR2 as shown in SEQ ID NO: 26, and
a VL-CDR3 as shown in SEQ ID NO: 3;
or
the heavy chain variable region comprises the following three complementarity determining regions (CDRs):
a VH-CDR1 as shown in SEQ ID NO: 33,
a VH-CDR2 as shown in SEQ ID NO: 54, and
a VH-CDR3 as shown in SEQ ID NO: 35;
the light chain variable region comprises the following three complementarity determining regions (CDRs):
a VL-CDR1 as shown in SEQ ID NO: 1,
a VL-CDR2 as shown in SEQ ID NO: 26, and
a VL-CDR3 as shown in SEQ ID NO: 3;
or
the heavy chain variable region comprises the following three complementarity determining regions (CDRs):
a VH-CDR1 as shown in SEQ ID NO: 33,
a VH-CDR2 as shown in SEQ ID NO: 54, and
a VH-CDR3 as shown in SEQ ID NO: 35;
the light chain variable region comprises the following three complementarity determining regions (CDRs):
a VL-CDR1 as shown in SEQ ID NO: 1,
a VL-CDR2 as shown in SEQ ID NO: 2, and
a VL-CDR3 as shown in SEQ ID NO: 3;
or
the heavy chain variable region comprises the following three complementarity determining regions (CDRs):
a VH-CDR1 as shown in SEQ ID NO: 33,
a VH-CDR2 as shown in SEQ ID NO: 34, and
a VH-CDR3 as shown in SEQ ID NO: 35;
the light chain variable region comprises the following three complementarity determining regions (CDRs):
a VL-CDR1 as shown in SEQ ID NO: 1,
a VL-CDR2 as shown in SEQ ID NO: 25, and
a VL-CDR3 as shown in SEQ ID NO: 3.

In another preferred embodiment, the heavy chain variable region of the antibody contains the amino acid sequence as shown in any one of SEQ ID NOs: 79-96; and/or the light chain variable region of the antibody contains the amino acid sequence as shown in any one of SEQ ID NOs: 61-78.

In another preferred embodiment, the antibody is a humanized antibody, and the heavy chain variable region of the antibody contains the amino acid sequence as shown in SEQ ID NO: 83; and the light chain variable region of the antibody contains the amino acid sequence as shown in SEQ ID NO: 64;
or, the heavy chain variable region of the antibody contains the amino acid sequence as shown in SEQ ID NO: 83; and the light chain variable region of the antibody contains the amino acid sequence as shown in SEQ ID NO: 62;
or, the heavy chain variable region of the antibody contains the amino acid sequence as shown in SEQ ID NO: 81; and the light chain variable region of the antibody contains the amino acid sequence as shown in SEQ ID NO: 63.

In another preferred embodiment, the amino acid sequence of the heavy chain variable region has at least 80%, 85%, 90%, 91%, 92%, 93%, 94%, 95%, 96%, 97%, 98% or 99% sequence homology or sequence identity with the amino acid sequence as shown in any one of SEQ ID NOs: 79-96 in the sequence listing.

In another preferred embodiment, the amino acid sequence of the light chain variable region has at least 80%, 85%, 90%, 91%, 92%, 93%, 94%, 95%, 96%, 97%, 98% or 99% sequence homology or sequence identity with the amino acid sequence as shown in any one of SEQ ID NOs: 61-78 in the sequence listing.

A recombinant protein comprising:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(ii) an optional tag sequence to assist in expression and/or purification.

In another preferred embodiment, the tag sequence comprises 6 His tag.

In another preferred embodiment, the recombinant protein (or polypeptide) comprises a fusion protein.

In another preferred embodiment, the recombinant protein is a monomer, dimer, or multimer.

In another aspect, it provides a chimeric antigen receptor (CAR) targeting B7-H3 whose antigen-binding domain comprises the heavy chain variable region according to the first aspect of the present invention or the light chain variable region according to the third aspect of the present invention.

In another preferred embodiment, the structure of the CAR is shown in the following Formula I:

L-scFv-H-TM-C-CD3ζ (I)

wherein,
each "-" is independently a linking peptide or a peptide bond;
L is absent or a signal peptide sequence;
H is absent or a hinge region;
TM is a transmembrane domain;
C is a co-stimulatory signaling molecule; and
CD3ζ is a cytoplasmic signal transduction sequence derived from CD3ζ.

In the seventh aspect of the present invention, it provides a polynucleotide encoding a polypeptide selected from the group consisting of:
(1) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(2) the recombinant protein according to the sixth aspect of the present invention.

In the eighth aspect of the present invention, it provides a vector comprising the polynucleotide according to the seventh aspect of the present invention.

In another preferred embodiment, the vector includes: a bacterial plasmid, bacteriophage, yeast plasmid, plant cell virus, and mammalian cell virus such as adenovirus, retrovirus, or other vectors.

In the ninth aspect of the present invention, it provides a genetically engineered host cell comprising the vector according to the eighth aspect of the present invention or the genome integrated with the polynucleotide according to the seventh aspect of the present invention.

In the tenth aspect of the present invention, it provides an antibody conjugate comprising:
(a) an antibody moiety, which is selected from the group consisting of the heavy chain variable region according to the first aspect of the present invention, the heavy chain according to the second aspect of the present invention, the light chain variable region according to the third aspect of the present invention, the light chain according to the fourth aspect of the present invention, and the antibody according to the fifth aspect of the present invention, and a combination thereof; and
(b) a coupling moiety coupled to the antibody moiety, which is selected from the group consisting of a detectable marker, a drug, a toxin, a cytokine, a radionuclide, an enzyme, and a combination thereof.

In another preferred embodiment, the antibody moiety is coupled to the coupling moiety by a chemical bond or a linker.

In another preferred embodiment, the antibody-drug conjugate (ADC) is shown in the following molecular formula: 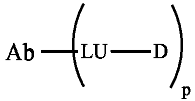 wherein
Ab is an anti-B7-H3 antibody,
LU is a linker;
D is a drug;
and the subscript p is a value selected from 1 to 10, preferably 1 to 8.

In another preferred embodiment, the drug is selected from the group consisting of a chemotherapeutic drug, a radiation therapeutic drug, a hormone therapeutic drug and an immunotherapeutic drug.

In another preferred embodiment, the toxin is selected from the group consisting of taxane, maytansinoid, auristatin, calicheamicin, anthracycline, docetaxel, cathepsin, ricin, gelonin. *Pseudomonas exotoxin,* diohtheria toxin, ribonuclease (RNase) and radioisotope.

In the eleventh aspect of the present invention, it provides an immune cell that expresses or is exposed outside the cell membrane with the antibody according to the fifth aspect of the present invention.

In another preferred embodiment, the immune cell comprises a NK cell, a T cell.

In another preferred embodiment, the immune cell is derived from humans or non-human mammals (such as mice).

In the twelfth aspect of the present invention, it provides a pharmaceutical composition comprising:
(i) an active ingredient, which is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof; and
(ii) a pharmaceutically acceptable carrier.

In another preferred embodiment, the pharmaceutical composition is a liquid formulation.

In another preferred embodiment, the pharmaceutical composition is an injection.

In another preferred embodiment, the pharmaceutical composition comprises 0.01-99.99% of the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof, and 0.01-99.99% of the pharmaceutical carrier, and the percentage is the mass percentage of the pharmaceutical composition.

In another preferred embodiment, the pharmaceutical composition further comprises a second active ingredient comprising a second antibody, or a chemotherapeutic agent.

In another preferred embodiment, the second antibody is selected from the group consisting of: a CTLA4 antibody, a PD-1 antibody, a PD-L1 antibody, a B7-H4 antibody, a HER2 antibody, a LAG3 antibody, a TIM-3 antibody, a 4-1BB antibody, a CD3 antibody and a TIGIT antibody.

In another preferred embodiment, the chemotherapeutic agent is selected from the group consisting of docetaxel, carboplatin, and a combination thereof.

In the thirteenth aspect of the present invention, it provides a use of an active ingredient selected from the group consisting of the heavy chain variable region according to the first aspect of the present invention, the heavy chain according to the second aspect of the present invention, the light chain variable region according to the third aspect of the present invention, the light chain according to the fourth aspect of the present invention, the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof, wherein the active ingredient is used for (a) preparing a drug for preventing and/or treating diseases related to abnormal expression or function of B7-H3; and/or (b) preparing a diagnostic reagent or kit.

In another preferred embodiment, the diagnostic reagent is a detective slip or a detection plate.

In another preferred embodiment, the disease related to the abnormal expression or function of B7-H3 is selected from the group consisting of tumors and autoimmune diseases.

In another preferred embodiment, the tumor is selected from the group consisting of melanoma, mesothelioma, non-small cell lung cancer, breast cancer, liver cancer, pancreatoesophageal cancer, adenocarcinoma, head and neck cancer, synovial sarcoma, colorectal cancer, kidney cancer, bladder cancer, prostate cancer, ovarian cancer, chronic hepatitis C virus infection, advanced solid cancer, malignant tumors of digestive organs, endometrial carcinoma, recurrent melanoma, head and neck squamous cell carcinoma, skin T-cell lymphoma, fallopian tube cancer, peritoneal tumor, muscle invasive bladder cancer, extensive stage small cell lung cancer, adult acute myeloid leukemia, atypical chronic myelogenous leukemia, epithelial ovarian cell carcinoma, B-cell chronic lymphocytic leukemia, skin B-cell non-Hodgkin's lymphoma, intraocular lymphoma, choriocarcinoma of testis, neuroblastoma, and esophageal cancer.

In another preferred example, the autoimmune disease is selected from the group consisting of systemic lupus erythematosus, oro-ocular Sjogren's syndrome, rheumatoid arthritis, ankylosing spondylitis, scleroderma, polyarteritis nodosa, Wegener granuloma, hyperthyroidism, insulin-dependent diabetes mellitus, myasthenia gravis, pemphigus vulgaris, pemphigoid, and transplant rejection.

In another preferred embodiment, the diagnostic reagent or kit is used for detecting B7-H3 protein in a sample.

In another preferred embodiment, the diagnostic reagent or kit is used for diagnosing B7-H3 related diseases.

In the fourteenth aspect of the present invention, it provides a method for detecting (including diagnostic or non-diagnostic) B7-H3 protein in a sample *in vitro,* which comprises the steps:
*(1) in vitro,* contacting the sample with the antibody according to the fifth aspect of the present invention;
(2) detecting the formation of an antigen-antibody complex, wherein the formation of a complex indicates the presence of B7-H3 protein in the sample.

In the fifteenth aspect of the present invention, it provides a composition for detecting B7-H3 protein in a sample *in vitro,* which comprises the antibody according to the fifth aspect of the present invention, the recombinant protein according to the sixth aspect of the present invention, the antibody conjugate according to the tenth aspect of the present invention, the immune cell according to the eleventh aspect of the present invention, and a combination thereof as an active ingredient.

In the sixteenth aspect of the present invention, it provides a detection plate comprising a substrate (support plate) and a test strip, and the test strip contains the antibody according to the fifth aspect of the present invention, the recombinant protein according to the sixth aspect of the present invention, and the antibody conjugate according to the tenth aspect of the present invention, the immune cell according to the eleventh aspect of the present invention, and a combination thereof.

In the seventeenth aspect of the present invention, it provides a kit comprising:
(1) a first container containing the antibody of the present invention; and/or
(2) a second container containing a secondary antibody against the antibody of the present invention;
   or,
   the kit contains the detection plate according to the sixteenth aspect of the present invention.

In the eighteenth aspect of the present invention, it provides a method for preparing a recombinant polypeptide, which comprises:
(a) culturing the host cell according to the ninth aspect of the present invention under conditions suitable for expression;
(b) isolating the recombinant polypeptide from the culture, the recombinant polypeptide being an antibody according to the fifth aspect of the present invention or a recombinant protein according to the sixth aspect of the present invention.

In the nineteenth aspect of the present invention, it provides a method for treating diseases related to abnormal expression or function of B7-H3, comprising administering to a subject in need an effective amount of the antibody according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, or a combination thereof.

In another preferred embodiment, the disease related to the abnormal expression or function of B7-H3 is cancer.

It should be understood that, within the scope of the present invention, the technical features specifically described above and below (such as the Examples) can be combined with each other, thereby constituting a new or preferred technical solution which needs not be described one by one.

### Description of Drawings

Figure 1 shows the relative tumor volume (mean ± standard error) of NCI-H1975 subcutaneous xenograft tumors.
Figure 2 shows the relative animal weight (mean ± standard error) of mice bearing NCI-H1975 subcutaneous xenograft tumors.

### Detailed Description

Through extensive and intensive studies, the inventors discovered an anti-B7-H3 antibody. The present invention also provides a preparing method and a use of the antibody. The antibody of the present invention has high affinity and selectivity for the target protein B7-H3, can be effectively endocytosed by cells and is easy to be expressed and purified. The ADC prepared by using the antibody of the present invention shows a significant tumor-inhibiting effect in animal models. On this basis, the present invention has been completed.

Specifically, in the present invention, B7-H3-his protein and CHO-K cells overexpressing B7-H3 are used, respectively, to immunize healthy Balb/c mice by subcutaneous and intraperitoneal multi-point injection. After immunization for 3-4 times, mice with the highest serum titer were euthanized and their spleens were taken to construct a phage display library. After liquid phase screening for three rounds, the ELISA plate was coated with B7-H3-His, and added with supernatant of anti-B7-H3 Fab. Positive clones that specifically bound to B7-H3 were obtained by ELISA screening. Positive clones were sequenced and performed with cell-level binding verification and monkey B7-H3 cross-binding verification. Full-length antibody construction was performed on candidate antibodies that can bind to both cell-level B7-H3 and monkey B7-H3.

Meanwhile, over 4,000 human PBMC samples were used to extract RNA, and primers for different germlines were designed according to the IMGT database. The scheme for constructing library was set according to the proportion of human genes reported in the literature, and a total of four ten-billion libraries with different germline proportions were formed, which were merged into a 100-billion-level antibody library. After liquid phase screening for three rounds, the ELISA plate was coated with Anti-Fd, and added with supernatant containing anti-B7-H3 Fab. Positive clones that specifically bound to B7-H3 were obtained by sandwich ELISA screening. The obtained positive clones were sequenced and the obtained monoclones were performed with verification of cell-level binding and monkey B7-H3 binding. Full-length antibody construction was performed on candidate antibodies that can bind to both cell-level B7-H3 and monkey B7-H3.

ExpiCHO-S cells were co-transfected with plasmids containing light and heavy chains of antibodies screened from the above murine immune library and human source library. The cell expression supernatant was collected after 7 days of expression, purified by Protein A affinity chromatography column, and the purity of the antibody was identified after purification by using SDS-PAGE and SEC.

Purified antibodies were subjected to protein-level binding, species cross-testing, cell-level binding and endocytosis or activity testing. Based on all experimental results, 8 candidate antibodies of the murine immune library and 3 of the human source library were finally obtained. Further, 2 of the candidate antibodies of the murine immune library were selected for humanization. The CDR of murine antibodies was transplanted into corresponding human antibody backbone by CDR transplantation. Then several pairs of light and heavy chain mutants were designed through 3D structure simulation, and the activity of the antibodies were verified after transient expression.

### B7-H3

During cellular immune response, the proliferation and activation of T cells require not only the first signal provided by T cell receptor (TCR) recognizing APC or MHC on the surface of tumor cells, but also the second signal provided by co-stimulatory molecules. The B7-CD28 superfamily is one of the currently discovered families of co-stimulatory molecules and belongs to the immunoglobulin superfamily. B7 family molecules provide stimulatory signals to enhance and maintain T cell immune responses, as well as inhibitory signals to limit and reduce T cell immune responses. Therefore, this family plays an important role in cancer diseases, organ transplantation and autoimmune diseases.

According to the function, the B7 family can be divided into 3 categories.

Group I: B7-1 (CD80) and B7-2 (CD86).

Group II: B7-H1 (PD-L1) and B7-DC (PD-L2).

Group III: consisting of B7-H3 (CD276) and B7-H4 (B7x). Their receptors have not been identified, but are deemed to be involved in co-stimulatory and co-inhibitory pathways.

B7-H3 (B7 homolog 3 protein), also known as CD276, is an important immune checkpoint molecule in the B7-CD28 family. It is a member of the B7 family identified by Chapoval et al. in human dendritic cell cDNA library in 2001. B7-H3 mainly exists in membrane protein and soluble form. Soluble B7-H3 (sB7-H3) is derived from membrane protein through metalloproteinase cleavage. In addition, B7-H3 protein is also found in exosomes and other extracellular vesicles.

B7-H3 is a T cell co-inhibitory molecule with partial co-stimulatory function. B7-H3 can effectively inhibit the function of T cells and NK cells, and also has an effect on bone development.

B7-H3 is expressed in various malignant tumors, and closely related to the growth, metastasis, recurrence and poor prognosis of malignant tumors. B7-H3 can downregulate T-helper type 1-mediated immune response to inhibit CD4+ T cell activation and inhibit cytokine production, thus potentially promoting immune escape in cancer cells.

B7-H3 is a type I transmembrane glycoprotein composed of 316 amino acids, with a molecular weight of 45-66 kDa, which has a similar molecular structure to B7-H1 (PD-L1). It contains a putative signaling peptide with 28 AA, a extracellular region with 217 AA consisting of immunoglobulin constant (IgC) and variable (IgV) structures, a transmembrane region, and a cytoplasmic domain with 45-amino acid. The B7-H3 gene consists of 10 exons, of which 4-7 exons encode the extracellular IgV-IgC domain.

It has been found at present that B7-H3 exists in two forms: 2Ig-B7-H3 and 4Ig-B7-H3. 2Ig-B7-H3 is expressed in murine and human cells, which has an extracellular IgV-IgC structure; 4Ig-B7-H3 is expressed only in human cells and consists of tandem repeats of IgV-IgC-IgV-IgC structures. The main form of human B7-H3 is 4IgB7-H3, located on chromosome 15. The mouse B7-H3 gene is located on chromosome 9. B7-H3 is one of the most evolutionarily conserved members in the B7 family, as it is commonly expressed in various species from teleost fish to mammals.

B7-H3 transcripts are widely expressed in tissues such as heart, liver, placenta, prostate, testes, uterus, pancreas, small intestine, colon and the like. The expression of B7-H3 protein is more limited to the cell surface, such as activated dendritic cells, monocytes, T cells, B cells, and NK cells.

B7-H3 are abnormally high expressed in various cancer cells or tissues, including gastric cancer, lung cancer, prostate cancer, kidney cancer, pancreatic cancer, ovarian cancer, breast cancer, endometrial cancer, liver cancer, colorectal cancer, oral cavity cancer, bladder cancer, osteosarcoma, and hematologic malignancies.

The molecular mechanisms regulating B7-H3 expression are unknown, but the expression of the B7-H3 protein is inversely proportional to miR-29 level, and the miR-29 binding site on B7-H3 is evolutionarily conserved.

### Antibody

As used herein, the term "antibody" or "immunoglobulin" is a heterotetrameric glycoprotein of about 150,000 Daltons with the same structural characteristics, which is composed of two identical light chains (L) and two identical heavy chains (H). Each light chain is connected to the heavy chain by a covalent disulfide bond, and the number of disulfide bonds between the heavy chains of different immunoglobulin isotypes is different. Each heavy and light chain also has regularly spaced intrachain disulfide bonds. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Special amino acid residues form an interface between the variable regions of the light and heavy chains.

As used herein, the term "variable" means that certain parts of the variable region of the antibody are different in sequence, which forms the binding and specificity of various specific antibodies to their specific antigens. However, the variability is not evenly distributed throughout the variable regions of antibodies. It is concentrated in three fragments called complementarity determining regions (CDR) or hypervariable regions in the variable regions of the light and heavy chains. The more conserved part of the variable region is called the framework region (FR). The variable regions of the natural heavy chain and light chain each contain four FR regions, which are roughly in a β -folded configuration, connected by three CDRs forming a connecting loop, and in some cases can form a partial β-folded structure. The CDRs in each chain are closely joined together by the FR region and form the antigen binding site of the antibody together with the CDRs of the other chain (see Kabat et al., NIH Publ. No. 91-3242, Volume I, pages 647-669 (1991)). Constant regions do not directly participate in the binding of antibodies to antigens, but they exhibit different effector functions, such as participating in antibody-dependent cytotoxicity of antibodies.

The "light chains" of vertebrate antibodies (immunoglobulins) can be classified into one of two distinct categories (called κ and λ) based on the amino acid sequence of their constant regions. According to the amino acid sequence of the constant region of their heavy chains, immunoglobulins can be divided into different types. There are mainly five classes of immunoglobulins: IgA, IgD, IgE, IgG and IgM, some of which can be further divided into subclasses (isotypes), such as IgG1, IgG2, IgG3, IgG4, IgA and IgA2. The heavy chain constant regions corresponding to different classes of immunoglobulins are called α, δ, ε, γ, and µ, respectively. The subunit structures and three-dimensional configurations of different classes of immunoglobulins are well known to those skilled in the art.

Generally, the antigen-binding properties of antibodies can be described by 3 specific regions located in the variable regions of the heavy and light chains, called variable regions (CDR), which divide this segment into 4 framework regions (FR). The amino acid sequences of the 4 FRs are relatively conservative and do not directly participate in the binding reaction. These CDRs form a circular structure, and the β-sheet formed by the FRs in between are close to each other in space structure. The CDRs on the heavy chain and the corresponding CDRs on the light chain constitute the antigen binding site of the antibody. The amino acid sequences of antibodies of the same type can be compared to determine which amino acids constitute the FR or CDR regions.

The present invention includes not only complete antibodies, but also fragments of antibodies with immunological activity or fusion proteins formed by antibodies and other sequences. Therefore, the present invention also includes fragments, derivatives and analogues of the antibodies.

In the present invention, antibodies include murine, chimeric, humanized or fully human antibodies prepared by techniques well known to those skilled in the art. Recombinant antibodies, such as chimeric and humanized monoclonal antibodies, including human and non-human parts, can be obtained by standard DNA recombination techniques, and they are all useful antibodies. A chimeric antibody is a molecule in which different parts are derived from different animal species, for example, a chimeric antibody having a variable region from a mouse monoclonal antibody and a constant region from a human immunoglobulin (see, for example, U.S. Patent Nos. 4,816,567 and U.S. Patent No. 4,816,397, which is hereby incorporated by reference in its entirety). Humanized antibodies refer to antibody molecules derived from non-human species, with one or more complementarity determining regions (CDRs) derived from non-human species and framework regions derived from human immunoglobulin molecules (see U.S. Patent 5,585,089, which is hereby incorporated by reference in its entirety). These chimeric and humanized monoclonal antibodies can be prepared using DNA recombination techniques well known in the art.

In the present invention, the antibody may be monospecific, bispecific, trispecific, or more multispecific.

In the present invention, the antibody of the present invention also includes a conservative variant thereof, which means that compared with the amino acid sequence of the antibody of the present invention, there are at most 10, preferably at most 8, more preferably at most 5, and most preferably at most 3 amino acids are replaced by amino acids with the same or similar properties to form a polypeptide. These conservatively variant polypeptides are preferably produced by amino acid substitutions according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Glv (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-B7-H3 antibody

The present invention provides an antibody against B7-H3 with high specificity and high-affinity, which comprises a heavy chain and a light chain. The heavy chain contains a heavy chain variable region (VH) amino acid sequence, and the light chain contains a light chain variable region (VL) amino acid sequence.

In another preferred embodiment, the antibody comprises a heavy chain variable region and a light chain variable region;
wherein the heavy chain variable region comprises the following three complementarity determining region (CDRs):

| VH-CDR1 Sequence Number | VH-CDR2 Sequence Number | VH-CDR3 Sequence Number |
|---|---|---|
| 33 | 34 | 35 |
| 33 | 54 | 35 |
| 97 | 54 | 35 |
| 36 | 37 | 38 |
| 36 | 55 | 56 |
| 39 | 40 | 41 |
| 57 | 40 | 41 |
| 42 | 43 | 44 |
| 45 | 46 | 47 |
| 48 | 49 | 50 |
| 51 | 52 | 53 |
| 51 | 52 | 58 |
| 51 | 59 | 60; |

and the light chain variable region comprises the following three complementarity determining region (CDRs):

| VL-CDR1 Sequence Number | VL-CDR2 Sequence Number | VL-CDR3 Sequence Number |
|---|---|---|
| 1 | 2 | 3 |
| 1 | 25 | 3 |
| 1 | 26 | 3 |
| 4 | 5 | 6 |
| 27 | 5 | 6 |
| 27 | 28 | 6 |
| 7 | 8 | 9 |
| 10 | 11 | 12 |
| 29 | 11 | 12 |
| 13 | 14 | 15 |
| 16 | 17 | 18 |
| 19 | 20 | 21 |
| 22 | 23 | 24 |
| 30 | 23 | 31 |
| 30 | 23 | 32. |

In another preferred embodiment, the heavy chain variable region of the antibody contains the amino acid sequence as shown in any one of SEQ ID NOs: 79-96; and/or the light chain variable region of the antibody contains the amino acid sequence as shown in any one of SEQ ID NOs: 61-78.

In another preferred embodiment, the antibody comprises:

| Name | Type | Heavy chain variable region | Light chain variable region |
|---|---|---|---|
| IL-P7-C05 | Unhumanized murine origin sequences | 79 | 61 |
| IL-P7-C05-H4L3 | Pair of humanized heavy and light chain sequences | 83 | 64 |
| IL-P7-C05-H4L1 | Pair of humanized heavy and light chain sequences | 83 | 62 |
| IL-P7-C05-H2L2 | Pair of humanized heavy and light chain sequences | 81 | 63 |
| IL-P7-C05-H2L1 | Pair of humanized heavy and light chain sequences | 81 | 62 |
| IL-P7-C05-H3L3 | Pair of humanized heavy and light chain sequences | 82 | 64 |
| IL-P7-C05-H1L1 | Pair of humanized heavy and light chain sequences | 80 | 62 |
| IL-P7-C05-H3L1 | Pair of humanized heavy and light chain sequences | 82 | 62 |
| IL-P5-C06 | Unhumanized murine origin sequences | 84 | 65 |
| IL-P5-C06-H2L2 | Pair of humanized heavy and light chain sequences | 86 | 67 |
| IL-P5-C06-H2L4 | Pair of humanized heavy and light chain sequences | 86 | 69 |
| IL-P5-C06-H2L3 | Pair of humanized heavy and light chain sequences | 86 | 68 |
| IL-P5-C06-H1L4 | Pair of humanized heavy and light chain sequences | 85 | 69 |
| IL-P5-C06-H1L1 | Pair of humanized heavy and light chain sequences | 85 | 66 |
| IL-P5-C06-H1L3 | Pair of humanized heavy and light chain sequences | 85 | 68 |
| IL-P5-C06-H3L3 | Pair of humanized heavy and light chain sequences | 87 | 68 |
| IL-P5-C06-H3L4 | Pair of humanized heavy and light chain sequences | 87 | 69 |
| IL-P6-D06 | Unhumanized murine origin sequences | 88 | 70 |
| IL-P6-C11 | Unhumanized murine origin sequences | 89 | 71 |
| IL-P6-B07 | Unhumanized murine origin sequences | 90 | 72 |
| IL-P6-C08 | Unhumanized murine origin sequences | 91 | 73 |
| IL-P5-B01 | Unhumanized murine origin sequences | 92 | 74 |
| IL-P7-A06 | Unhumanized murine origin sequences | 93 | 75 |
| NL24-A17 | Sequences screened from human source library | 94 | 76 |
| NL24-A68 | Sequences screened from human source library | 95 | 77 |
| NL24-A10 | Sequences screened from human source library | 96 | 78. |

In a preferred embodiment, the antibody comprises a heavy chain variable region and a light chain variable region; wherein
the heavy chain variable region comprises the following three complementarity determining region (CDRs):
a VH-CDR1 as shown in SEQ ID NO: 97,
a VH-CDR2 as shown in SEQ ID NO: 54, and
a VH-CDR3 as shown in SEQ ID NO: 35;
the light chain variable region comprises the following three complementarity determining region (CDRs):
a VL-CDR1 as shown in SEQ ID NO: 1,
a VL-CDR2 as shown in SEQ ID NO: 26, and
a VL-CDR3 as shown in SEQ ID NO: 3;
or
the heavy chain variable region comprises the following three complementarity determining region (CDRs):
a VH-CDR1 as shown in SEQ ID NO: 97,
a VH-CDR2 as shown in SEQ ID NO: 54, and
a VH-CDR3 as shown in SEQ ID NO: 35;
the light chain variable region comprises the following three complementarity determining region CDRs:
a VL-CDR1 as shown in SEQ ID NO: 1,
a VL-CDR2 as shown in SEQ ID NO: 2, and
a VL-CDR3 as shown in SEQ ID NO: 3;
or
the heavy chain variable region comprises the following three complementarity determining region (CDRs):
a VH-CDR1 as shown in SEQ ID NO: 33,
a VH-CDR2 as shown in SEQ ID NO: 34, and
a VH-CDR3 as shown in SEQ ID NO: 35;
the light chain variable region comprises the following three complementarity determining region (CDRs):
a VL-CDR1 as shown in SEQ ID NO: 1,
a VL-CDR2 as shown in SEQ ID NO: 25, and
a VL-CDR3 as shown in SEQ ID NO: 3.

In another preferred embodiment, the antibody is a humanized antibody, and the heavy chain variable region of the antibody contains the amino acid sequence as shown in SEQ ID NO: 83; and the light chain variable region of the antibody contains the amino acid sequence as shown in SEQ ID NO: 64;
or, the heavy chain variable region of the antibody contains the amino acid sequence as shown in SEQ ID NO: 83; and the light chain variable region of the antibody contains the amino acid sequence as shown in SEQ ID NO: 62;
or, the heavy chain variable region of the antibody contains the amino acid sequence as shown in SEQ ID NO: 81; and the light chain variable region of the antibody contains the amino acid sequence as shown in SEQ ID NO: 63.

In another preferred embodiment, any one of the above amino acid sequences further includes a derivative sequence that is optionally with at least one amino acid added, deleted, modified, and/or substituted and can retain the binding affinity of B7-H3.

In another preferred embodiment, the sequence formed by adding, deleting, modifying and/or substituting at least one amino acid sequence is preferably an amino acid sequence with homology or sequence identity of at least 80%, preferably at least 85%, more preferably, at least 90%, and most preferably at least 95%. More preferably, the number of added, deleted, modified and/or substituted amino acids may be 1-7, more preferably 1-5, more preferably 1-3, more preferably 1-2.

The antibody of the present invention may be a double-chain or single-chain antibody, and may be selected from the group consisting of an animal-derived antibody, a chimeric antibody, a humanized antibody, more preferably a humanized antibody, a human-animal chimeric antibody, more preferably a fully humanized antibody.

The antibody derivative of the present invention may be a single-chain antibody, and/or an antibody fragment, such as Fab, Fab', (Fab')2 or other known antibody derivatives in the field and the like, and any one or more of IgA, IgD, IgE, IgG and IgM antibodies or antibodies of other subtypes.

Wherein the animal is preferably a mammal, such as a mouse.

The antibody of the present invention may be a chimeric antibody, a humanized antibody, a CDR grafted and/or modified antibody targeting B7-H3 (such as human B7-H3).

### Antibody preparation

The sequence of the DNA molecule of the antibody or its fragment of the present invention can be obtained by conventional techniques, such as PCR amplification or genomic library screening. In addition, the coding sequences of the light chain and the heavy chain can also be fused together to form a single chain antibody.

Once the relevant sequences are obtained, the relevant sequences can be obtained in large quantities by recombination method. It is usually cloned into a vector, then transferred into a cell, and then the relevant sequence is isolated from the host cell after proliferation by conventional methods.

In addition, the relevant sequences can also be synthesized by artificial synthesis, especially when the fragment length is short. Usually, by first synthesizing multiple small fragments, and then ligating to obtain a very long fragment.

At present, the DNA sequence encoding the antibody (or fragment or derivative thereof) of the present invention can be obtained completely through chemical synthesis. The DNA sequence can then be introduced into various existing DNA molecules (or such as vectors) and cells known in the art. In addition, mutations can also be introduced into the protein sequence of the present invention through chemical synthesis.

The present invention also relates to a vector containing the above-mentioned appropriate DNA sequence and an appropriate promoter or control sequence. These vectors can be used to transform appropriate host cells so that they can express proteins.

The host cell may be a prokaryotic cell, such as a bacterial cell; or a lower eukaryotic cell, such as a yeast cell; or a higher eukaryotic cell, such as a mammalian cell. Preferred animal cells include (but are not limited to): CHO-S, HEK-293 cells.

Generally, the transformed host cell is cultured under conditions suitable for expression of the antibody of the present invention. Then the antibody of the present invention is purified by conventional immunoglobulin purification steps, such as protein A-Sepharose, hydroxyapatite chromatography, gel electrophoresis, dialysis, ion exchange chromatography, hydrophobic chromatography, molecular sieve chromatography or affinity chromatography and other conventional separation and purification methods well known to those skilled in the art.

The obtained monoclonal antibody can be identified by conventional means. For example, the binding specificity of the monoclonal antibody can be determined by immunoprecipitation or *in vitro* binding assays (such as radioimmunoassay (RIA) or enzyme-linked immunosorbent assay (ELISA)). The binding affinity of the monoclonal antibody can be determined, for example, by the Scatchard analysis of Munson et al., Anal. Biochem., 107:220 (1980).

The antibody of the present invention can be expressed in the cell, on the cell membrane, or secreted out of the cell. If necessary, the physical, chemical, and other characteristics can be used to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultrasonic treatment, ultracentrifugation, molecular sieve chromatography (gel filtration), adsorption chromatography, ion exchange chromatography, high performance liquid chromatography (HPLC) and various other liquid chromatography techniques and combinations of these methods.

### Antibody-Drug conjugate (ADC)

The present invention also provides antibody-drug conjugate (ADC) based on the antibody of the present invention.

Typically, the antibody-drug conjugate comprises the antibody and an effector molecule, wherein the antibody being coupled to the effector molecule, and preferably chemically coupled. Wherein the effector molecule is preferably a drug having therapeutic activity. In addition, the effector molecule may be one or more of a toxic protein, a chemotherapeutic drug, a small molecule drug or a radionuclide.

The antibody of the present invention can be coupled with the effector molecule by a coupling agent. Examples of the coupling agent may be any one or more of a non-selective coupling agent, a coupling agent using a carboxyl group, a peptide chain, and a coupling agent using a disulfide bond. The non-selective coupling agent refers to a compound that makes the effector molecule and the antibody form a covalent bond, such as glutaraldehyde. The coupling agent using carboxyl groups can be any one or more of cis-aconitic acid anhydride coupling agents (such as cis-aconitic acid anhydride) and acyl hydrazone coupling agents (the coupling site is acyl hydrazone).

Certain residues on the antibody (such as Cys or Lys, etc.) are used to connect to a variety of functional groups, including an imaging reagent (such as a chromophores and a fluorescent group), a diagnostic reagent (such as a MRI contrast agent and a radioisotope), a stabilizer (such as glycol polymer) and a therapeutic agent. The antibody can be conjugated to the functional agent to form an antibody-functional agent conjugate. The functional agent (e.g., a drug, a detection reagent, a stabilizer) is coupled (covalently linked) to the antibody. The functional agent may be directly or indirectly linked to the antibody through a linker.

Antibodies can be conjugated with drugs to form antibody-drug conjugates (ADCs). Typically, the ADC contains a linker between the drug and the antibody. The linker may be a degradable or a non-degradable linker. A degradable linker is typically easily degraded in the intracellular environment. For example, the linker is degraded at the target site, so that the drug is released from the antibody. Suitable degradable linkers include, for example, an enzymatically degraded linker, including a peptidyl-containing linker that can be degraded by intracellular proteases (such as lysosomal proteases or endosomal proteases), or a sugar linker, for example, a glucuronide-containing linker that can be degraded by glucuronidase. The peptidyl linker may include, for example, a dipeptide such as valine-citrulline, phenylalanine-lysine or valine-alanine. Other suitable degradable linkers include, for example, a pH-sensitive linker (for example, a linker that is hydrolyzed at a pH of less than 5.5, such as a hydrazone linker) and linkers that degrade under reducing conditions (for example, a disulfide bond linker). A non-degradable linker typically releases the drug under conditions where the antibody is hydrolyzed by a protease.

Before being connected to the antibody, the linker has a reactive group capable of reacting with certain amino acid residues, and the connection is achieved through the reactive group. Sulfhydryl-specific reactive groups are preferred and include, for example, maleimide compounds, halogenated amides (such as iodine, bromine, or chloro); halogenated esters (such as iodine, bromine, or chloro); halogenated methyl ketones (such as iodine, bromine or chloro), benzyl halides (such as iodine, bromine or chloro); vinyl sulfone, pyridyl disulfide; mercury derivatives such as 3,6-Di-(mercury methyl) dioxane, and the counter ion is acetate, chloride or nitrate; and polymethylene dimethyl sulfide thiosulfonate. The linker may include, for example, maleimide linked to the antibody via thiosuccinimide.

The drug may be any cytotoxic, inhibiting cell growth or immunosuppressive drug. In embodiments, the linker connects the antibody and the drug, and the drug has a functional group that can be bonded to the linker. For example, the drug may have an amino group, a carboxyl group, a sulfhydryl group, a hydroxyl group, or a ketone group that can form a bond with the linker. In the case where the drug is directly connected to the linker, the drug has a reactive active group before being connected to the antibody.

In another preferred embodiment, the drug is selected from the group consisting of a chemotherapeutic drug, a radiation therapeutic drug, a hormone therapeutic drug and an immunotherapeutic drug. Specifically, useful drug categories include, for example, anti-tubulin drugs, DNA minor groove binding reagents, DNA replication inhibitors, alkylating reagents, antibiotics, folate antagonists, antimetabolites, chemotherapy sensitizers, topoisomerase inhibitors, Vinca Alkaloids, etc.. Examples of particularly useful cytotoxic drugs include, for example, DNA minor groove binding reagents, DNA alkylating reagents, and tubulin inhibitors. Typical cytotoxic drugs include, for example, auristatins, camptothecins, duocarmycins, etoposides, maytansines and maytansinoids (e.g., DM1 and DM4), taxanes, benzodiazepines or benzodiazepine containing drugs (e.g., pyrrolo[1,4] benzodiazepines (PBDs), indolinobenzodiazepines and oxazolidinobenzodiazepines and vinca alkaloids.

The toxins preferred in the present invention are selected from the group consisting of taxane, maytansinoid, auristatin, calicheamicin, anthracycline, docetaxel, cathepsin, ricin, gelonin, *Pseudomonas exotoxin,* diphtheria toxin, ribonuclease (RNase) and radioisotope.

In the present invention, the drug-linker can be used to form ADC in one simple step. In other embodiments, bifunctional linker compounds can be used to form ADC in a two-step or multi-step process. For example, the cysteine residue reacts with the reactive part of the linker in the first step, and in the subsequent step, the functional group on the linker reacts with the drug to form ADC.

Generally, the functional group on the linker is selected to facilitate the specific reaction with the appropriate reactive group on the drug moiety. As a non-limiting example, the azide-based moiety can be used to specifically react with the reactive alkynyl group on the drug moiety. The drug is covalently bound to the linker through the 1,3-dipolar cycloaddition between the azide and alkynyl groups. Other useful functional groups include, for example, ketones and aldehydes (suitable for reacting with hydrazides and alkoxyamines), phosphines (suitable for reacting with azides); isocyanates and isothiocyanates (suitable for reaction with amines and alcohols); and activated esters, such as N-hydroxysuccinimide ester (suitable for reaction with amines and alcohols). These and other ligation strategies, such as those described in Bioconjugation Technology, Second Edition (Elsevier), are well known to those skilled in the art. Those skilled in the art can understand that for the selective reaction between the drug moiety and the linker, when a complementary pair of reactive functional groups is selected, each member of the complementary pair can be used for both the linker and drug.

The present invention also provides a method for preparing ADC, which may further include: combining an antibody with a drug-linker compound under conditions sufficient to form an antibody-drug conjugate (ADC).

In certain embodiments, the method of the present invention includes combining the antibody with a bifunctional linker compound under conditions sufficient to form an antibody-linker conjugate. In these embodiments, the method of the present invention further includes: combining the antibody linker conjugate to the drug moiety under conditions sufficient to covalently link the drug moiety to the antibody via a linker.

In some embodiment, the antibody-drug conjugate ADC is shown in the following molecular formula: 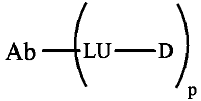 wherein
Ab is an antibody,
LU is a linker;
D is a drug;
and the subscript p is a value selected from 1 to 8.

### Use

The present invention also provides the use of the antibody, antibody-drug conjugate ADC, recombinant protein, and/or immune cell of the present invention, for example, for preparing diagnostic preparations or preparing medicines.

Preferably, the drug is a drug for preventing and/or treating diseases related to abnormal expression or function of B7-H3.

The uses of the antibody, ADC, recombinant protein, and/or immune cell of the present invention include (but are not limited to):
(i) diagnosis, prevention and/or treatment of tumor occurrence, growth and/or metastasis, especially tumors with high B7-H3 expression. The tumors include, but are not limited to, melanoma, mesothelioma, non-small cell lung cancer, breast cancer, liver cancer, synovial sarcoma, metastatic colon cancer, kidney cancer, bladder cancer, prostate cancer, ovarian cancer, chronic hepatitis C virus infection, advanced solid cancer, malignant tumors of digestive organs, endometrial carcinoma, recurrent melanoma, head and neck squamous cell carcinoma, skin T-cell lymphoma, fallopian tube cancer, peritoneal tumor, muscle invasive bladder cancer, extensive stage small cell lung cancer, adult acute myeloid leukemia, atypical chronic myelogenous leukemia, epithelial ovarian cell carcinoma, B-cell chronic lymphocytic leukemia, skin B-cell non-Hodgkin's lymphoma, intraocular lymphoma, choriocarcinoma of testis, neuroblastoma, and esophageal cancer.
(ii) diagnosis, prevention and/or treatment of autoimmune diseases, wherein the autoimmune diseases include, but are not limited to, systemic lupus erythematosus, oro-ocular Sjogren's syndrome, rheumatoid arthritis, ankylosing spondylitis, scleroderma, polyarteritis nodosa, Wegener granuloma, hyperthyroidism, insulin-dependent diabetes mellitus, myasthenia gravis, pemphigus vulgaris, pemphigoid, and transplant rejection.

### Detection use and kit

The antibody or ADC of the present invention can be used in detection applications, for example, to detect samples, so as to provide diagnostic information.

In the present invention, the samples (specimen) used include cells, tissue samples and biopsy specimens. The term "biopsy" used in the present invention shall include all kinds of biopsy known to those skilled in the art. Therefore, the biopsy used in the present invention may include, for example, excision samples of tumors, tissue samples prepared by endoscopic methods or organ puncture or needle biopsy.

The samples used in the present invention include fixed or preserved cell or tissue samples.

The present invention also provides a kit containing the antibody (or fragment thereof) of the present invention. In a preferred embodiment of the present invention, the kit further includes a container, instructions for use, buffers, and the like. In a preferred embodiment, the antibody of the present invention can be immobilized on a detection plate.

### Pharmaceutical composition

The present invention also provides a composition. In a preferred embodiment, the composition is a pharmaceutical composition, which contains the above-mentioned antibody or active fragment or fusion protein or ADC thereof or corresponding immune cell, and a pharmaceutically acceptable carrier. Generally, these substances can be formulated in a non-toxic, inert and pharmaceutically acceptable aqueous carrier medium, wherein the pH is usually about 5-8, preferably about 6-8, although the pH value can vary with the nature of the substance being formulated and the condition to be treated.

The formulated pharmaceutical composition can be administered by conventional routes, including (but not limited to): intratumoral, intraperitoneal, intravenous, or topical administration. Typically, the administrating route of the pharmaceutical composition of the present invention is preferably injection or oral administration. The injection preferably includes intravenous injection, intramuscular injection, intraperitoneal injection, intradermal injection, or subcutaneous injection. The pharmaceutical composition is a variety of conventional dosage forms in the art, preferably in the form of solid, semi-solid or liquid, and may be an aqueous solution, non-aqueous solution or suspension, and more preferably a tablet, capsule, or granule, injection or infusion, etc.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention is a pharmaceutical composition for preventing and/or treating diseases related to abnormal expression or function of B7-H3.

The pharmaceutical composition of the present invention can be directly used to bind B7-H3 protein molecules, and thus can be used to prevent and treat tumors and other diseases.

The pharmaceutical composition of the present invention contains a safe and effective amount (such as 0.001-99wt%, preferably 0.01-90wt%, more preferably 0.1-80wt%) of the above-mentioned monoclonal antibody (or conjugate thereof) of the present invention and a pharmaceutical acceptable carrier or excipient. Such carriers include (but are not limited to): saline, buffer, glucose, water, glycerol, ethanol, and a combination thereof. The pharmaceutical preparation should match the mode of administration. The pharmaceutical composition of the present invention can be prepared in the form of injections, for example, it can be prepared by conventional methods with physiological saline or an aqueous solution containing glucose and other adjuvants. Pharmaceutical compositions such as injections and solutions should be manufactured under aseptic conditions. The dosage of the active ingredient is a therapeutically effective amount, for example, about 1 microgram/kg body weight to about 5 mg/kg body weight per day. In addition, the polypeptides of the present invention can also be used together with other therapeutic agents.

In the present invention, preferably, the pharmaceutical composition of the present invention further includes one or more pharmaceutical carriers. The pharmaceutical carrier is a conventional pharmaceutical carrier in the art, and the pharmaceutical carrier can be any suitable physiologically or pharmaceutically acceptable pharmaceutical excipient. The pharmaceutical excipients are conventional pharmaceutical excipients in the field, and preferably include pharmaceutically acceptable excipients, fillers or diluents. More preferably, the pharmaceutical composition includes 0.01-99.99% of the aforementioned protein and 0.01-99.99% of a pharmaceutical carrier, and the percentage is a mass percentage of the pharmaceutical composition.

In the present invention, preferably, the administration amount of the pharmaceutical composition is an effective amount, and the effective amount is an amount capable of alleviating or delaying the progression of the disease, degenerative or traumatic condition. The effective amount can be determined on an individual basis and will be partly based on consideration of the symptoms to be treated and the results sought. Those skilled in the art can determine the effective amount by using the aforementioned factors such as individual basis and using no more than conventional experiments.

When using the pharmaceutical composition, a safe and effective amount of the immunoconjugate is administered to the mammal, wherein the safe and effective amount is usually at least about 10 micrograms/kg body weight, and in most cases, it does not exceed about 50 mg/kg body weight, preferably the dosage is about 10 micrograms/kg body weight to about 20 mg/kg body weight. Of course, the specific dosage should also consider factors such as the route of administration, the patient's health status, etc., which are within the skill range of a skilled physician.

The present invention provides the application of the above-mentioned pharmaceutical composition in the preparation of drugs for preventing and/or treating diseases related to abnormal expression or function of B7-H3. Preferably, the disease related to the abnormal expression or function of B7-H3 is cancer and autoimmune disease.

### The main advantages of the present invention include:

(a) The antibody of the present invention has high affinity and selectivity for the target protein B7-H3.
(b) The antibody of the present invention can be effectively endocytosed by cells after binding to target protein in cell experiment.
(c) The antibody of the present invention is easy to be expressed and purified.
(d) After prepared to ADC, the antibody of the present invention shows a significant tumor-inhibiting effect with a single administration in tumor-bearing animal models.

The present invention will be further illustrated below with reference to the specific examples. It is to be understood that these examples are for illustrative purposes only and are not intended to limit the scope of the invention. For the experimental methods in the following examples, in which the specific conditions are not specifically indicated, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified. Unless indicated otherwise, the parts and percentage are weight parts and weight percentage.

### Example 1 Immunization of Mouse

Healthy Balb/c mice were selected to be immunized every two weeks by subcutaneous and intraperitoneal multi-point injection. One group of mice were immunized with B7-H3his protein and the other group with CHOK cells overexpressing B7-H3. The first immunization was performed with Freund's complete adjuvant, followed by Freund's incomplete adjuvant. After immunization for 3-4 times, the serum titers of mice were assayed by ELISA. Mice with the highest titer were sacrificed and their spleens were taken to construct a phage display library.

### Example 2 Construction of murine immune library and screening

After immunization, the spleen of mice with qualified titer was taken, the total RNA of the mouse spleens was extracted with chloroform. Then the cDNA library was prepared by reverse transcription PCR. The light chain and heavy chain variable region genes of mice were amplified by combined primers (Sanyou Bio), and the combined Fab fragments were inserted into phage vector to construct the Fab antibody library. The library was prepared into a phage display library. B7-H3hisbiotin was coated to magnetic beads for three rounds of liquid phase screening. After liquid phase screening for three rounds, the ELISA plate was coated with B7-H3-His, and then added with supernatant containing anti-B7-H3 Fab. Positive clones that specifically bound to B7-H3 were obtained by ELISA screening.

The obtained positive clones were sequenced and the obtained monoclones were performed with verification of cell-based binding and monkey B7-H3 binding. Full-length antibody construction was performed on candidate antibodies that can bind to both cell-level B7-H3 and monkey B7-H3.

Names and sequence numbers of part of the antibodies constructed in the present example are shown in Table 1.

**Table.1 Murine antibodies and sequence numbers thereof**

| Antibody | Amino acid sequence number of heavy chain variable region | Amino acid sequence number of light chain variable region |
|---|---|---|
| IL-P7-C05 | 79 | 61 |
| IL-P5-C06 | 84 | 65 |
| IL-P6-D06 | 88 | 70 |
| IL-P6-C11 | 89 | 71 |
| IL-P6-B07 | 90 | 72 |
| IL-P6-C08 | 91 | 73 |
| IL-P5-B01 | 92 | 74 |
| IL-P7-A06 | 93 | 75 |

### Example 3 Construction of fully human natural immune library and screening

Construction of Sanyou's 100-billion-level antibody library: over 4,000 human PBMC samples were used to extract RNA, and primers for different germlines were designed according to IMGT database. The scheme for constructing library was set according to the proportion of human genes reported in the literature, and a total of four ten-billion-level libraries with different germline proportions were formed, which were merged into a 100-billion-level antibody library.

B7-H3hisbiotin was coated to magnetic beads for three rounds of liquid phase screening. After liquid phase screening for three rounds, the ELISA plate was coated with AntiFd, then added with supernatant containing anti-B7-H3 Fab, incubated for 1 h, then added with 2 µg/mL of B7-H3Hisbiotin and incubated for 1 hour, and finally the signal was detected with NeutrAvidin-HRP. Positive clones that specifically bound to B7-H3 were obtained by sandwich ELISA screening.

The obtained positive clones were sequenced and the obtained monoclones were performed with verification of cell-based binding and monkey B7-H3 binding. Full-length antibody construction was performed on candidate antibodies that can bind to both cell-level B7-H3 and monkey B7-H3.

Names and sequence numbers of part of the antibodies constructed in the present example are shown in Table 2.

**Table.2 Fully Human antibodies and sequence numbers thereof**

| Antibody | Amino acid sequence number of heavy chain variable region | Amino acid sequence number of light chain variable region |
|---|---|---|
| NL24A17HC | 76 | 94 |
| NL24A68HC | 77 | 95 |
| NE24A10HC | 78 | 96 |

### Example 4 Expression and purification of full-length antibody, and humanization of murine antibody

ExpiCHOS cells were co-transfected with plasmids containing light and heavy chains of antibodies. The cell expression supernatant was collected after 7 days of expression, purified by Protein A affinity chromatography column. For specific experimental procedures, refer to the AKTA User Manual. The purified antibodies were replaced into PBS buffer at PH7.2 by dialysis. A total of 23 antibodies from the murine immune library and 39 antibodies from the human source library were expressed and purified, and finally a total of 57 antibodies were successfully expressed and purified, of which 19 were from murine immune library and 38 were from human source library.

The results shows that the specific antibodies listed in Examples 2 and 3 were successfully expressed in ExpiCHOS cells.

The CDR of murine antibodies (IL-P7-C05, IL-P5-C06) were transplanted into corresponding human antibody backbone by CDR transplantation. Then several pairs of light and heavy chain mutants were designed through 3D structure simulation, and the activity of the antibodies were verified after transient expression.

The heavy chain and light chain variable regions of part of the antibodies constructed in the present example and sequence numbers thereof are shown in Table 3.

**Table.3 Humanized heavy chain and light chain variable regions and sequence numbers thereof**

| Heavy chain variable region | Amino acid sequence number | Light chain variable region | Amino acid sequence number |
|---|---|---|---|
| ILP7C05huVH1 | 80 | ILP7C05huVL1 | 62 |
| ILP7C05huVH2 | 81 | ILP7C05huVL2 | 63 |
| ILP7C05huVH3 | 82 | ILP7C05huVL3 | 64 |
| ILP7C05huVH4 | 83 | ILP5C06huVL1 | 66 |
| ILP5C06huVH1 | 85 | ILP5C06huVL2 | 67 |
| ILP5C06huVH2 | 86 | ILP5C06huVL3 | 68 |
| ILP5C06huVH3 | 87 | ILP5C06huVL4 | 69 |

The above heavy chain variable region and light chain variable region can be arbitrarily combined to form a new humanized antibody.

### Example 5 Protein level binding and species cross-reactivity assay

Human B7-H3-his, mouse B7-H3-his and monkey B7-H3-his were diluted to 2 µg/mL with PBS, and added to the ELISA plate at 30 µL per well. The ELISA plate was then incubated in a refrigerator at 4°C for 16-20 hours. The next day, the ELISA plate was washed once with a lotion (PBS containing 0.05% Tween 20) and then blocked with 5% skim milk powder for 1 hour. After blocking, the ELISA plate was washed once, then added with gradient-diluted anti-B7-H3 antibodies, and incubated at room temperature for 1 h. After incubated, the ELISA plate was washed three times, then added with goat anti-human HRP-labeled secondary antibody, and incubated at room temperature for 1 h. After incubated, the ELISA plate was washed three times, then developed for 5-15 minutes by adding TMB, and finally terminated with 2M sulfuric acid. The data were recorded on the microplate reader.

The results of species cross-reactivity are shown in Table 4, showing that almost all antibodies showed cross-reactivity between human and monkey B7-H3, and most of them showed cross-reactivity between human and mouse B7-H3.

Subsequently, the humanized antibody constructed in Example 4 and some murine antibodies and fully human antibodies were verified again for binding to human B7-H3 at protein level. The results are shown in Table 5, in which all the listed antibodies had a high affinity to human B7-H3.

**Table.4 B7-H3 protein-binding species cross-reactivity**

| Species | Human | Monkey | Mouse |
|---|---|---|---|
| Name | B7-H3 protein-binding activity EC₅₀ (µg/mL) | | |
| IL-P7-C05 | 0.07882 | 0.14 | 1.206 |
| IL-P5-C06 | 0.1775 | 0.1156 | 0.1343 |
| IL-P6-D06 | 0.1741 | 0.1189 | 1.13 |
| IL-P6-C11 | 0.2302 | 0.01519 | 0.5232 |
| IL-P6-B07 | 0.1318 | 0.1829 | 1.289 |
| IL-P6-C08 | 0.04242 | 0.05409 | No significant binding |
| IL-P5-B01 | 0.137 | 0.1151 | No significant binding |
| IL-P7-A06 | 0.1949 | No significant binding | No significant binding |
| NL24-A17 | 0.2259 | 0.0527 | 0.06426 |
| NL24-A68 | 0.5374 | 0.131 | 0.205 |
| NL24-A10 | 0.296 | 0.1245 | 0.2192 |

**Table.5 Binding activity of antibodies to human B7-H3 at protein level**

| Name | EC₅₀ of protein-binding activity (µg/mL) |
|---|---|
| IL-P7-C05 | 0.044 |
| IL-P7-C05-H4L3 | 0.03953 |
| IL-P7-C05-H4L1 | 0.035 |
| IL-P7-C05-H2L2 | 0.051 |
| IL-P7-C05-H2L1 | 0.111 |
| IL-P7-C05-H3L3 | 0.071 |
| IL-P7-C05-H1L1 | 0.053 |
| IL-P7-C05-H3L1 | 0.026 |
| IL-P5-C06 | 0.302 |
| IL-P5-C06-H2L2 | 0.147 |
| IL-P5-C06-H2L4 | 0.223 |
| IL-P5-C06-H2L3 | 0.151 |
| IL-P5-C06-H1L4 | 0.237 |
| IL-P5-C06-H1L1 | 0.206 |
| IL-P5-C06-H1L3 | 0.164 |
| IL-P5-C06-H3L3 | 0.151 |
| IL-P5-C06-H3L4 | 0.156 |
| IL-P6-D06 | 0.233 |
| IL-P6-C11 | 0.182 |
| IL-P6-B07 | 0.194 |
| IL-P6-C08 | 0.088 |
| IL-P5-B01 | 0.248 |
| IL-P7-A06 | 0.172 |
| NL24-A17 | 0.280 |
| NL24-A68 | 0.285 |
| NL24-A10 | 0.355 |

### Example 6 Cell level binding assay

CHOK cells overexpressing B7-H3 or NCI-H322 cells endogenously expressing B7-H3 were added to a round-bottom 96-well plate at 100,000 cells per well, centrifuged to remove the supernatant, and then added with a gradient-diluted anti-B7-H3 antibody and incubated at 4 degrees for 1 h. After incubation, the cells were washed twice with FACS buffer (PBS containing 2% FBS), then added with fluorescent-labeled goat anti-human secondary antibody (Abcam, ab98596) and incubated for 0.5 h. After incubation, the cells were washed twice with FACS buffer, then resuspended with FACS buffer and detected with flow cytometry (Beckman, cytoFlex).

The results are shown in Table 6. Antibodies listed in the table have high binding capacity against both CHO cells that artificially express B7-H3 and human non-small cell lung cancer cells (NCI-H322) with high endogenous B7-H3 level.

**Table.6 Cell-based binding activity of antibodies**

| Name | EC₅₀ of CHO cell-binding activity (µg/mL) | EC₅₀ of NCI-H322-binding activity (µg/mL) |
|---|---|---|
| IL-P7-C05 | 0.487 | 0.0126 |
| IL-P7-C05-H4L3 | 0.480 | 0.0137 |
| IL-P7-C05-H4L1 | 0.468 | 0.0138 |
| IL-P7-C05-H2L2 | 0.455 | 0.0145 |
| IL-P7-C05-H2L1 | 0.426 | 0.0144 |
| IL-P7-C05-H3L3 | 0.431 | 0.0106 |
| IL-P7-C05-H1L1 | 0.426 | 0.0107 |
| IL-P7-C05-H3L1 | 0.454 | 0.0109 |
| IL-P5-C06 | 0.954 | 0.0795 |
| IL-P5-C06-H2L2 | 1.119 | 0.0919 |
| IL-P5-C06-H2L4 | 0.747 | 0.0576 |
| IL-P5-C06-H2L3 | 0.826 | 0.0618 |
| IL-P5-C06-H1L4 | 0.596 | 0.0436 |
| IL-P5-C06-H1L1 | 1.051 | 0.0895 |
| IL-P5-C06-H1L3 | 0.941 | 0.0829 |
| IL-P5-C06-H3L3 | 1.179 | 0.137 |
| IL-P5-C06-H3L4 | 1.133 | 0.107 |
| IL-P6-D06 | 0.245 | 0.103 |
| IL-P6-C11 | 0.311 | 0.210 |
| IL-P6-B07 | - | 0.249 |
| IL-P6-C08 | - | 0.158 |
| IL-P5-B01 | 0.430 | 0.131 |
| IL-P7-A06 | 0.278 | 0.072 |
| NL24-A17 | 0.665 | 0.02669 |
| NL24-A68 | - | 0.0484 |
| NL24-A10 | 0.448 | 0.03036 |

### Example 7 Detection of internalization activity of candidate antibodies

CHOK cells overexpressing B7-H3 or NCI-H322 cells endogenously expressing B7-H3 were seeded into 96-well plates and cultured overnight in an incubator at 37 °C. The antibody against B7-H3 was labeled with a toxin-containing secondary antibody (ATS, IT51), then added to the cells and cultured in an incubator at 37 °C for 3 days. Along with the endocytosis of the antibodies, the toxins marked on the antibody entered the cell, causing toxicity to the cell and reducing the cell viability. Finally the viability of the cell was detected by the cytotoxicity detection kit (Promega, G3581).

The results are shown in Table 7. All the listed antibodies can be effectively endocytosed by B7-H3-expressing CHO cells, and some selected antibodies further showed efficient endocytic activities in the endocytosis experiment of human non-small cell lung cancer cells (NCI-H322).

**Table.7 Endocytic activity of candidate antibodies**

| Name | IC₅₀ of CHO endocytosis experiment (µg/mL) | IC₅₀ of NCI-H322 endocytosis experiment (µg/mL) |
|---|---|---|
| IL-P7-C05 | 0.0144 | 0.00296 |
| IL-P7-C05-H4L3 | 0.0199 | 0.002535 |
| IL-P5-C06 | 0.0209 | 0.002505 |
| IL-P6-D06 | - | 0.00179 |
| IL-P6-C11 | 0.0035 | - |
| IL-P5-B01 | 0.0028 | - |
| IL-P7-A06 | 0.0086 | - |
| NL24-A68 | 0.0035 | - |

### Example 10 Evaluation of pharmacodynamics in vitro

MMAE (Monomethyl auristatin E) was used as the coupling effector molecule, and the antibody IL-P7-C05-H4L3 prepared above was used to construct the antibody-drug conjugate IL-P7-C05-H4L3-MMAE for evaluation of pharmacodynamics *in vitro.*

The CellTiter-Glo method was used to detect the inhibitory activity of IL-P7-C05-H4L3-MMAE, after incubated for 72 hours, against B7-H3-positive human tumor cell lines NCI-H1975 and BxPC-3, and B7-H3 negative human tumor cell line Raji.

The results are shown in Table 8. IL-P7-C05-H4L3-MMAE has strong killing ability against tumor cells highly expressing B7-H3 (NCI-H1975 and BxPC-3), and poor killing activity against B7-H3-negative tumor cells.

**Table.8 Inhibitory IC₅₀ value of IL-P7-C05-H4L3-MMAE incubated for 72 h against the proliferation of human tumor cell lines**

| Cell line | Type | IC₅₀ (µM) |
|---|---|---|
| NCI-H1975 | Human non-small cell lung cancer | 0.0074 |
| BxPC-3 | Human pancreatic cancer | 0.0077 |
| Raji | Human lymphoma | 0.0341 |

The CellTiter-Glo method was used to detect the inhibitory activity of IL-P7-C05-H4L3-MMAE and the isotype control Isotype-MMAE, after incubated for 5 days, against B7-H3 positive human tumor cell lines Calu-6 and BxPC-3, and B7-H3 negative human tumor cell line Raji.

The results are shown in Table 9. IL-P7-C05-H4L3-MMAE showed strong killing activity against B7-H3-high tumor cells (Calu-6 and BxPC-3), and poor killing activity against B7-H3-negative tumor cells. The Isotype-MMAE control has much lower cell-killing activity than IL-P7-C05-H4L3-MMAE.

**Table.9 Inhibitory IC₅₀ value of IL-P7-C05-H4L3-MMAE and Isotype-MMAE incubated for 5 days against the proliferation of human tumor cell lines**

| Cell line | Type | MHB008-MNAE IC₅₀ (µM) | Isotype-MMAE IC₅₀ (µM) |
|---|---|---|---|
| Calu-6 | Human lung undifferentiated carcinoma | 0.0003 | 0.0604 |
| BxPC-3 | Human pancreatic cancer | 0.0055 | 0.0567 |
| Raji | Human lymphoma | 0.1054 | 0.3027 |

### Example 11 Evaluation of pharmacodynamics in vivo

The inhibitory effect of single administration of IL-P7-C05-H4L3-MMAE and isotype control Isotype-MMAE on tumor growth was detected in BALB/c nude mice models that were subcutaneous transplanted with human non-small cell lung cancer NCI-H1975 cells.

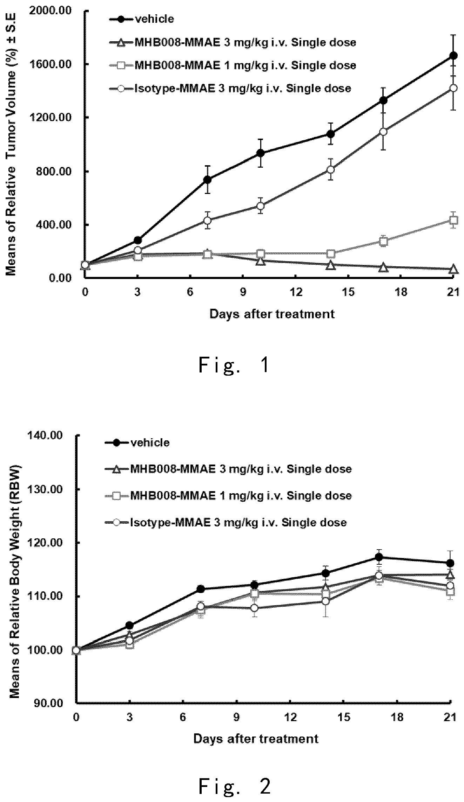

Tumor volumes and animal weights are shown in Figures 1 and 2.

The results showed that a single administration of IL-P7-C05-H4L3-MMAE via tail vein at doses of both 1 mg/kg and 3 mg/kg had a significant inhibitory effect on the growth of subcutaneous transplanted NCI-H1975 tumors (P <0.01). The inhibition rates (TGI) on tumor growth at the end of the experiment (21 days after administration) were 78.4% and 101.8%, respectively, and the relative tumor proliferation rates (%T/C) of the two dose groups were 26.2% and 4.2%, respectively. In addition, in the group of 3 mg/kg dose, the tumor showed atrophy and regression. After a single administration of the isotype control Isotype-MMAE via the tail vein at a dose of 3 mg/kg, there was no significant difference in tumor growth as compared with the vehicle control group (P>0.05). The single administration of IL-P7-C05-H4L3-MMAE at the dose of 1 mg/kg and 3 mg/kg, and the single administration of isotype control Isotype-MMAE at the dose of 3 mg/kg had no significant effect on the change of animal weight. There were no significant clinical abnormalities, and the animals tolerated well.

All literatures mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

Sequences involved in the present application

### Light chain variable region CDRs

| Name | CDR1 | | CDR2 | | CDR3 | |
|---|---|---|---|---|---|---|
| | Sequence | SEQ ID NO: | Sequence | SEQ ID NO: | Sequence | SEQ ID NO: |
| ILP7C05 | RASENIYSNLA | 1 | AATNLA D | 2 | QHFWGTPPW T | 3 |
| ILP7C05huVL1 | RASENIYSNLA | 1 | AATNLA D | 2 | QHFWGTPPW T | 3 |
| ILP7C05huVL2 | RASENIYSNLA | 1 | AATNLQS | 25 | QHFWGTPPW T | 3 |
| ILP7C05huVL3 | RASENIYSNLA | 1 | AASNLQS | 26 | QHFWGTPPW T | 3 |
| ILP5C06 | SASSSISSNYL H | 4 | STSNLAS | 5 | QQYSGYPLT | 6 |
| ILPSC06huVL1 | RASSSISSNYL H | 27 | STSNLAS | 5 | QQYSGYPLT | 6 |
| ILP5C06huVL2 | RASSSISSNYL H | 27 | STSNLAS | 5 | QQYSGYPLT | 6 |
| ILP5C06huVL3 | RASSSISSNYL H | 27 | STSNLAS | 5 | QQYSGYPLT | 6 |
| ILP5C06huVL4 | RASSSISSNYL H | 27 | STSNLQS | 28 | QQYSGYPLT | 6 |
| ILP6D06 | KASQNVGTNV A | 7 | SASYRYS | 8 | QQYNSYPYT | 9 |
| ILP6C11 | SASSSISYMH | 10 | DTSKLAS | 11 | QQWSSNPLT | 12 |
| ILP6B07 | SASSSVSYMH | 29 | DTSKLAS | 11 | QQWSSNPLT | 12 |
| ILP6C08 | RASENIYSYLA | 13 | NAKTLAE | 14 | QHHYGTPPY T | 15 |
| ILP5B01 | KASQNVGTNV A | 16 | SASYRYS | 17 | QQYNSYPYT | 18 |
| ILP7A06 | KASQNVGTAV A | 19 | SASNRYT | 20 | QQYSSYPFT | 21 |
| NL24A17LC | RASQSVSTYL A | 22 | DASNRAT | 23 | QQRSNWPPL FT | 24 |
| NL24A68LC | RASQSVSSYL A | 30 | DASNRAT | 23 | QQRSNWPPS LT | 31 |
| NL24A10LC | RASQSVSSYL A | 30 | DASNRAT | 23 | QQRSNWPPM YT | 32 |

### Heavy chain variable region CDRs

| Name | CDR1 | | CDR2 | | CDR3 | |
|---|---|---|---|---|---|---|
| | Sequence | SEQ ID NO: | Sequence | SEQ ID NO: | Sequence | SEQ ID NO: |
| ILP7C05 | GFTFSSYGM S | 33 | TINSNGGTT Y | 34 | NEEFRRGLA Y | 35 |
| ILP7C05huVH1 | GFTFSSYGM S | 33 | TINSNGGTT Y | 34 | NEEFRRGLA Y | 35 |
| ILP7C05huVH2 | GFTFSSYGM S | 33 | TINSNGGTT Y | 34 | NEEFRRGLA Y | 35 |
| ILP7C05huVH3 | GFTFSSYGM S | 33 | AINSNGGST Y | 54 | NEEFRRGLA Y | 35 |
| ILP7C05huVH4 | GFTFSSYAM S | 97 | AINSNGGST Y | 54 | NEEFRRGLA Y | 35 |
| ILP5C06 | GYTFTDYAI H | 36 | IINTYNGNS N | 37 | NYGSSFNWY FDV | 38 |
| ILPSC06huVH1 | GYTFTDYAI H | 36 | IINTYNGNS N | 37 | NYGSSFNWY FDV | 38 |
| ILP5C06huVH2 | GYTFTDYAI H | 36 | IINTYNGNS N | 37 | NYGSSFNWY FDV | 38 |
| ILP5C06huVH3 | GYTFTDYAI H | 36 | IINTYNGNS N | 37 | NYGSSFNWY FDV | 38 |
| ILP6D06 | GYTFTDYAI H | 36 | VINTYSGNT N | 55 | GVTYGSGYP YWYFDV | 56 |
| ILP6C11 | GYTFTDYVI H | 39 | GINRDSGGT T | 40 | WKFPWYFDV | 41 |
| ILP6B07 | GYTFTEYVI H | 57 | GINRDSGGT T | 40 | WKFPWYFDV | 41 |
| ILP6C08 | GFTFSSYGM S | 42 | TISGGGRYT Y | 43 | HYDGYLDY | 44 |
| ILP5B01 | GDSITSGSW N | 45 | YISYSGSIY | 46 | GNPAWFAY | 47 |
| ILP7A06 | GYTFTDYA MH | 48 | IINTYNGNT N | 49 | SRGPWDRYF DV | 50 |
| NL24A17HC | GGTFSSYAI | 51 | GIIPILGIAN | 52 | GDSGYDSNY | 53 |
| | S | | | | | |
| NL24A68HC | GGTFSSYAI S | 51 | GIIPILGIAN | 52 | GGSGRYLPDY | 58 |
| NL24A10HC | GGTFSSYAI S | 51 | GIIPIFGTAN | 59 | GERGSYLIDY | 60 |

### Light chain variable region

| Type | Name | Sequence | SE Q ID NO |
|---|---|---|---|
| Murine light chain variable region | ILP7C05 | DIQMIQSPASLSVSVGETVTITCRASENIYSNLAW YQQKQGKSPQLLVYAATNLADGVPSRFSGSGSG TQYSLKINSLQSEDFGSYYCQHFWGTPPWTFGGG TKLEIK | 61 |
| Humanized light chain variable region | ILP7C05hu VL1 | DIQMTQSPSSLSASVGDRVTITCRASENIYSNLAW YQQKPGKAPKLLVYAATNLADGVPSRFSGSGSG TDYTLTISSLQPEDFATYYCQHFWGTPPWTFGQG TKLEIK | 62 |
| Humanized light chain variable region | ILP7C05hu VL2 | DIQMTQSPSSLSASVGDRVTITCRASENIYSNLAW YQQKPGKAPKLLVYAATNLQSGVPSRFSGSGSG TDYTLTISSLQPEDFATYYCQHFWGTPPWTFGQG TKLEIK | 63 |
| Humanized light chain variable region | ILP7C05hu VL3 | DIQMTQSPSSLSASVGDRVTITCRASENIYSNLAW YQQKPGKAPKLLIYAASNLQSGVPSRFSGSGSGT DYTLTISSLQPEDFATYYCQHFWGTPPWTFGQGT KLEIK | 64 |
| Murine light chain variable region | ILP5C06 | DIVLTQSPTTMAASPGEKITITCSASSSISSNYLHW YQQKPGFSPKLLIYSTSNLASGVPARFSGSGSGTS YSLTISSVEAEDAATYYCQQYSGYPLTFGAGTKL ELK | 65 |
| Humanized light chain variable region | ILP5C06hu VL1 | DIQLTQSPSSMSASVGDRITITCRASSSISSNYLHW YQQKPGKSPKLLIYSTSNLASGVPSRFSGSGSGTD YTLTISSVQPEDFATYYCQQYSGYPLTFGQGTKL ELK | 66 |
| Humanized light chain variable region | ILP5C06hu VL2 | DIQLTQSPSSLSASVGDRITITCRASSSISSNYLHW YQQKPGKAPKLLIYSTSNLASGVPSRFSGSGSGT DFTLTISSVQPEDFATYYCQQYSGYPLTFGQGTK LELK | 67 |

| | | | |
|---|---|---|---|
| Humanized light chain variable region | ILP5C06hu VL3 | DIQLTQSPSSLSASVGDRVTITCRASSSISSNYLH WYQQKPGKAPKLLIYSTSNLASGVPSRFSGSGSG TDFTLTISSLQPEDFATYYCQQYSGYPLTFGQGTK LELK | 68 |
| Humanized light chain variable region | ILP5C06hu VL4 | DIQMTQSPSSLSASVGDRVTITCRASSSISSNYLH WYQQKPGKAPKLLIYSTSNLQSGVPSRFSGSGSG TDFTLTISSLQPEDFATYYCQQYSGYPLTFGQGTK LELK | 69 |
| Murine light chain variable region | ILP6D06 | DIVMTQSQKFMSTSVGDRVSVTCKASQNVGTNV AWYQQKPGQSPKALIYSASYRYSGVPDRFTGSGS GTDFTLTISNVQSEDLAEYFCQQYNSYPYTFGGG TKLEIK | 70 |
| Murine light chain variable region | ILP6C 11 | DIELTQSPAIMSASPGEKVTMTCSASSSISYMHW YQQKPGTSPKRWIYDTSKLASGVPARFSGSGSGT SYSLTISRVEAEDAATYYCQQWSSNPLTFGSGTK LEIK | 71 |
| Murine light chain variable region | ILP6B07 | DIVLTQSPAIMSASPGEKVTMTCSASSSVSYMHW YQQKSGTSPKRWIYDTSKLASGVPARFSGSGSGT SYSLTISSMEAEDAATYYCQQWSSNPLTFGAGTK LELK | 72 |
| Murine light chain variable region | ILP6C08 | DIQMIQSPASLSASVGETVTITCRASENIYSYLAW YQQKQGKSPQLLVYNAKTLAEGVPSRFSGSGSG TQFSLKINSLQPEDFGSYYCQHHYGTPPYTFGGG TKLEIK | 73 |
| Murine light chain variable region | ILP5B01 | DIVMTQSQKFMSTSVGDRVSVTCKASQNVGTNV AWYQQKPGQSPKALIYSASYRYSGVPDRFTGSGS GTDFTLTISNVQSEDLAEYFCQQYNSYPYTFGGG TKLEIK | 74 |
| Murine light chain variable region | ILP7A06 | DIVMTQSQKFMSTSVGDRVSVTCKASQNVGTAV AWYQQKPGQSPKLLIYSASNRYTGVPDRFTGSGS GTDFTLTISNMQSEDLADYFCQQYSSYPFTFGSG TKLEIK | 75 |
| Light chain variable region screened from human source library | NL24A17L C | EIVLTQSPATLSLSPGERATLSCRASQSVSTYLAW YQQKPGQAPRLLIYDASNRATGIPARFSGSGSGT DFTLTISSLEPEDYAVYYCQQRSNWPPLFTFGPGT KVDIK | 76 |

| | | | |
|---|---|---|---|
| Light chain variable region screened from human source library | NL24A68L C | EIVLTQSPATLSLSPGERATLSCRASQSVSSYLAW YQQKPGQAPRLLIYDASNRATGIPARFSGSGSGT DFTLTISSLEPEDFAVYYCQQRSNWPPSLTFGGGT KVEIK | 77 |
| Light chain variable region screened from human source library | NL24A10L C | EIVLTQSPATLSLSPGERATLSCRASQSVSSYLAW YQQKPGQAPRLLIYDASNRATGIPARFSGSGSGT DFTLTISSLEPEDFAVYYCQQRSNWPPMYTFGQG TKLEIK | 78 |

### Heavy chain variable region

| Type | Name | Sequence | SEQ ID NO |
|---|---|---|---|
| Murine heavy chain variable region | ILP7C05 | EVQLVESGGDLVQPGGSLKLSCAASGFTFSSYGM SWVRQTPDKRLELVATINSNGGTTYYPDSVKGRF TISRDNAKHTLYLQMSSLKSEDTAMYYCTRNEEF RRGLAYWGQGTLVTVSA | 79 |
| Humanized heavy chain variable region | ILP7C05hu VH1 | EVQLVESGGGLVQPGGSLRLSCAASGFTFSSYGM SWVRQAPGKGLELVATINSNGGTTYYPDSVKGR FTISRDNAKHTLYLQMSSLRAEDTAVYYCTRNEE FRRGLAYWGQGTLVTVSS | 80 |
| Humanized heavy chain variable region | ILP7C05hu VH2 | EVQLVESGGGLVQPGGSLRLSCAASGFTFSSYGM SWVRQAPGKGLELVATINSNGGTTYYADSVKGR FTISRDNAKNTLYLQMSSLRAEDTAVYYCTRNEE FRRGLAYWGQGTLVTVSS | 81 |
| Humanized heavy chain variable region | ILP7C05hu VH3 | EVQLVESGGGLVQPGGSLRLSCAASGFTFSSYGM SWVRQAPGKGLELVAAINSNGGSTYYADSVKGR FTISRDNAKNTLYLQMSSLRAEDTAVYYCTRNEE FRRGLAYWGQGTLVTVSS | 82 |
| Humanized heavy chain variable region | ILP7C05hu VH4 | EVQLVESGGGLVQPGGSLRLSCAASGFTFSSYAM SWVRQAPGKGLELVAAINSNGGSTYYADSVKGR FTISRDNAKNTLYLQMSSLRAEDTAVYYCTRNEE FRRGLAYWGQGTLVTVSS | 83 |
| Murine heavy chain variable region | ILP5C06 | QVQLQQSGPELVRPGVSVKISCKGSGYTFTDYAI HWAKQGHAKSLEWIGIINTYNGNSNYNQNFKGK ATMTVDKSSSTAYMELTRLTSEDSAIYYCARNY GSSFNWYFDVWGQGTTLTVSS | 84 |
| Humanized heavy chain variable region | ILP5C06hu VH1 | QVQLVQSGAEVKKPGASVKVSCKGSGYTFTDYA IHWARQAPGQGLEWIGIINTYNGNSNYAQKFQG RATMTVDKSTSTAYMELSRLRSEDTAVYYCARN YGSSFNWYFDVWGQGTTVTVSS | 85 |
| Humanized heavy chain variable region | ILP5C06hu VH2 | QVQLVQSGAEVKKPGASVKVSCKGSGYTFTDYA IHWGRQAPGQGLEWIGIINTYNGNSNYAQKFQG RATMTRDTSTSTAYMELSRLRSEDTAVYYCARN YGSSFNWYFDVWGQGTTVTVSS | 86 |
| Humanized heavy chain variable region | ILP5C06hu VH3 | QVQLVQSGAEVKKPGASVKVSCKASGYTFTDYA IHWGRQAPGQGLEWMGIINTYNGNSNYAQKFQG RVTMTRDTSTSTAYMELSRLRSEDTAVYYCARN YGSSFNWYFDVWGQGTTVTVSS | 87 |
| Murine heavy chain variable region | ILP6D06 | QVQLQQSGPELVRPGVSVKISCKGSGYTFTDYAI HWVKQSHAKSLEWIGVINTYSGNTNYNQKFKGR ATMTVDKSSSTAYMELARLTSEDSAIYFCARGVT YGSGYPYWYFDVWGAGTTVTVSS | 88 |
| Murine heavy chain variable region | ILP6C 11 | EVQLQQSGPELVKPGASVRISCKTSGYTFTDYVI HWVKQNHGGSLEWIGGINRDSGGTTYNQKFQD KVTLTVDKSSNTAYMELRSLTSEDSAVYYCTKW KFPWYFDVWGAGTTVTVSS | 89 |
| Murine heavy chain variable region | ILP6B07 | EVQLQQSGPELVKPGASVRISCKTSGYTFTEYVIH WVKQNHGGSLEWIGGINRDSGGTTYNQKFQDK VTLTVDKSSNTAYMELRSLTSEDSAVYYCTKWK FPWYFDVWGAGTTVTVSS | 90 |
| Murine heavy chain variable region | ILP6C08 | EVKLVESGGDLVKPGGSLKLSCAASGFTFSSYGM SWVRQTSDKRLEWVATISGGGRYTYYPDSVKGR FTISRDSSKNTLYLQMSSLKSEDTAMYYCVRHYD GYLDYWGLGTTLTVSS | 91 |
| Murine heavy chain variable region | ILP5B01 | EVQLQESGPSLVKPSQTLSLTCSVTGDSITSGSWN WIRKFPGNKLEYMGYISYSGSIYYNPSLKSRISITR DTSKNQYYLQLNSVTTEDTATYYCARGNPAWFA YWGQGTLVTVSA | 92 |
| Murine heavy chain variable region | ILP7A06 | QVQLQQSGPEVVRPGVSVKISCKGSGYTFTDYA MHWVKQSHAKSLEWIGIINTYNGNTNYNQKFKG KATMTVDKSSSTAYMEFARLTSEDSAIYYCARSR | 93 |
| | | GPWDRYFDVWGAGTTVTVSS | |
| Heavy chain variable region screened from human source library | NL24A17H C | QVQLVQSGAEVKKPGSSVKVSCKASGGTFSSYAI SWVRQAPGQGLEWMGGIIPILGIANYAQKFQGR VTITADESTSTAYMELSSLRSEDTAVYYCARGDS GYDSNYWGQGTLVTVSA | 94 |
| Heavy chain variable region screened from human source library | NL24A68H C | QVQLVQSGAEVKKPGSSVKVSCKASGGTFSSYAI SWVRQAPGQGLEWMGGIIPILGIANYAQKFQGR VTITADKSTSTAYMELSSLRAEDTAVYYCARGGS GRYLPDYWGQGTLVTVSA | 95 |
| Heavy chain variable region screened from human source library | NL24A10H C | EVQLVQSGAEVKKPGSSVKVSCKASGGTFSSYAI SWVRQAPGQGLEWMGGIIPIFGTANYAQKFQGR VTITADESTSTAYMELSSLRSEDTAVYYCARGER GSYLIDYWGQGTLVTVSA | 96 |

## Claims

1. A heavy chain variable region of an antibody, comprising the following three complementarity determining region (CDRs):
a VH-CDR1 as shown in SEQ ID NO: 3n,
a VH-CDR2 as shown in SEQ ID NO: 3n+1, and
a VH-CDR3 as shown in SEQ ID NO: 3n+2;
wherein each n is independently 11, 12, 13, 14, 15, 16, or 17;
or, wherein the heavy chain variable region comprises the following three complementarity determining region (CDRs):
a VH-CDR1 as shown in SEQ ID NO: 97,
a VH-CDR2 as shown in SEQ ID NO: 54, and
a VH-CDR3 as shown in SEQ ID NO: 35;
or
a VH-CDR1 as shown in SEQ ID NO: 33,
a VH-CDR2 as shown in SEQ ID NO: 54, and
a VH-CDR3 as shown in SEQ ID NO: 35;
or
a VH-CDR1 as shown in SEQ ID NO: 36,
a VH-CDR2 as shown in SEQ ID NO: 55, and
a VH-CDR3 as shown in SEQ ID NO: 56;
or
a VH-CDR1 as shown in SEQ ID NO: 57,
a VH-CDR2 as shown in SEQ ID NO: 40, and
a VH-CDR3 as shown in SEQ ID NO: 41;
or
a VH-CDR1 as shown in SEQ ID NO: 51,
a VH-CDR2 as shown in SEQ ID NO: 52, and
a VH-CDR3 as shown in SEQ ID NO: 58;
or
a VH-CDR1 as shown in SEQ ID NO: 51,
a VH-CDR2 as shown in SEQ ID NO: 59, and
a VH-CDR3 as shown in SEQ ID NO: 60;
wherein any one of the above amino acid sequences further includes a derivative sequence that is optionally with at least one amino acid added, deleted, modified, and/or substituted and can retain the binding affinity to B7-H3.

2. A heavy chain of an antibody comprising the heavy chain variable region according to claim 1.

3. A light chain variable region of an antibody, comprising the following three complementarity determining region (CDRs):
a VL-CDR1 as shown in SEQ ID NO: 3m+1,
a VL-CDR2 as shown in SEQ ID NO: 3m+2, and
a VL-CDR3 as shown in SEQ ID NO: 3m+3;
wherein each m is independently 0, 1, 2, 3, 4, 5, 6, or 7;
or, wherein the light chain variable region comprises the following three complementarity determining region (CDRs):
a VL-CDR1 as shown in SEQ ID NO: 1,
a VL-CDR2 as shown in SEQ ID NO: 25, and
a VL-CDR3 as shown in SEQ ID NO: 3;
or
a VL-CDR1 as shown in SEQ ID NO: 1,
a VL-CDR2 as shown in SEQ ID NO: 26, and
a VL-CDR3 as shown in SEQ ID NO: 3;
or
a VL-CDR1 as shown in SEQ ID NO: 27,
a VL-CDR2 as shown in SEQ ID NO: 5, and
a VL-CDR3 as shown in SEQ ID NO: 6;
or
a VL-CDR1 as shown in SEQ ID NO: 27,
a VL-CDR2 as shown in SEQ ID NO: 28, and
a VL-CDR3 as shown in SEQ ID NO: 6;
or
a VL-CDR1 as shown in SEQ ID NO: 29,
a VL-CDR2 as shown in SEQ ID NO: 11, and
a VL-CDR3 as shown in SEQ ID NO: 12;
or
a VL-CDR1 as shown in SEQ ID NO: 30,
a VL-CDR2 as shown in SEQ ID NO: 23, and
a VL-CDR3 as shown in SEQ ID NO: 31;
or
a VL-CDR1 as shown in SEQ ID NO: 30,
a VL-CDR2 as shown in SEQ ID NO: 23, and
a VL-CDR3 as shown in SEQ ID NO: 32;
wherein any one of the above amino acid sequences further includes a derivative sequence that is optionally with at least one amino acid added, deleted, modified, and/or substituted and can retain the binding affinity to B7-H3.

4. A light chain of an antibody comprising the light chain variable region according to claim 3.

5. An antibody comprising:
(1) the heavy chain variable region according to claim 1; and/or
(2) the light chain variable region according to claim 3;
or the antibody has the heavy chain according to claim 2; and/or the light chain according to claim 4,
wherein any one of the above amino acid sequences further includes a derivative sequence that is optionally with at least one amino acid added, deleted, modified, and/or substituted and can retain the binding affinity to B7-H3.

6. The antibody according to claim 5 comprising a heavy chain variable region according to claim 1 and a light chain variable region according to claim 3;
wherein the heavy chain variable region and the light chain variable region are as follows:
| VH Sequence Number | VL Sequence Number |
|---|---|
| 79 | 61 |
| 83 | 64 |
| 83 | 62 |
| 81 | 63 |
| 81 | 62 |
| 82 | 64 |
| 80 | 62 |
| 82 | 62 |
| 84 | 65 |
| 86 | 67 |
| 86 | 69 |
| 86 | 68 |
| 85 | 69 |
| 85 | 66 |
| 85 | 68 |
| 87 | 68 |
| 87 | 69 |
| 88 | 70 |
| 89 | 71 |
| 90 | 72 |
| 91 | 73 |
| 92 | 74 |
| 93 | 75 |
| 94 | 76 |
| 95 | 77 |
| 96 | 78 |
wherein any one of the above amino acid sequences further includes a derivative sequence that is optionally with at least one amino acid added, deleted, modified, and/or substituted and can retain the binding affinity to B7-H3.

7. The antibody according to claim 5 comprising a heavy chain variable region according to claim 1 and a light chain variable region according to claim 3; wherein
the heavy chain variable region comprises the following three complementarity determining region (CDRs):
a VH-CDR1 as shown in SEQ ID NO: 97,
a VH-CDR2 as shown in SEQ ID NO: 54, and
a VH-CDR3 as shown in SEQ ID NO: 35;
the light chain variable region comprises the following three complementarity determining region (CDRs):
a VL-CDR1 as shown in SEQ ID NO: 1,
a VL-CDR2 as shown in SEQ ID NO: 26, and
a VL-CDR3 as shown in SEQ ID NO: 3;
or
the heavy chain variable region comprises the following three complementarity determining region (CDRs):
a VH-CDR1 as shown in SEQ ID NO: 97,
a VH-CDR2 as shown in SEQ ID NO: 54, and
a VH-CDR3 as shown in SEQ ID NO: 35;
the light chain variable region comprises the following three complementarity determining region (CDRs):
a VL-CDR1 as shown in SEQ ID NO: 1,
a VL-CDR2 as shown in SEQ ID NO: 2, and
a VL-CDR3 as shown in SEQ ID NO: 3;
or
the heavy chain variable region comprises the following three complementarity determining region (CDRs):
a VH-CDR1 as shown in SEQ ID NO: 33,
a VH-CDR2 as shown in SEQ ID NO: 34, and
a VH-CDR3 as shown in SEQ ID NO: 35;
the light chain variable region comprises the following three complementarity determining region (CDRs):
a VL-CDR1 as shown in SEQ ID NO: 1,
a VL-CDR2 as shown in SEQ ID NO: 25, and
a VL-CDR3 as shown in SEQ ID NO: 3.

8. A recombinant protein comprising:
(i) the heavy chain variable region according to claim 1, the heavy chain according to claim 2, the light chain variable region according to claim 3, the light chain according to claim 4, or the antibody according to any one of claims 5-7; and
(ii) an optional tag sequence to assist in expression and/or purification.

9. An antibody conjugate comprising:
(a) an antibody moiety, which is selected from the group consisting of the heavy chain variable region according to claim 1, the heavy chain according to claim 2, the light chain variable region according to claim 3, the light chain according to claim 4, and the antibody according to any one of claims 5-7, and a combination thereof; and
(b) a coupling moiety coupled to the antibody moiety, which is selected from the group consisting of a detectable marker, a drug, a toxin, a cytokine, a radionuclide, an enzyme, and a combination thereof.

10. Use of an active ingredient, which is selected from the group consisting of the heavy chain variable region according to claim 1, the heavy chain according to claim 2, the light chain variable region according to claim 3, the light chain according to claim 4, the antibody according to any one of claims 5-7, the recombinant protein according to claim 8, the antibody conjugate according to claim 9, and a combination thereof, wherein the active ingredient is used for (a) preparing a diagnostic reagent or kit; and/or (b) preparing a drug for preventing and/or treating diseases related to abnormal expression or function of B7-H3.
